# EUROPEAN PATENT SPECIFICATION

(11) **EP 1 007 090 B1**
(45) Date of publication and mention of the grant of the patent: **30.12.2009**
(21) Application number: 97913955.7
(22) Date of filing: 29.10.1997
(51) Int. Cl.: A61K 39/395, C07K 16/18, C07K 16/28

(54) **IDENTIFICATION OF UNIQUE BINDING INTERACTIONS BETWEEN CERTAIN ANTIBODIES AND THE HUMAN B7.1 (CD80) AND B7.2 (CD28) CO-STIMULATORY ANTIGENS**
IDENTIFIKATION VON BINDUNGS- INTERAKTIONEN ZWISCHEN GEWISSEN ANTIKORPERN UND DEN HUMANEN COSTIMULATORISCHEN ANTIGENEN B7.1 (CD80) UND B7.2 (CD28)
IDENTIFICATION D'INTERACTIONS DE LIAISON UNIQUES ENTRE CERTAINS ANTICORPS ET LES ANTIGENES COSTIMULANTS B7.1 (CD80) ET B7.2 (cd28) HUMAINS

(30) Priority: 08.11.1996 US 746361
(43) Date of publication of application: 14.06.2000
(60) Divisional application: 07013357.4
(73) Proprietor: Biogen Idec Inc., Cambridge, MA 02142 (US)
(72) Inventor: ANDERSON, Darrell, R., Escondido, CA 92029 (US); HANNA, Nabil, Rancho Santa Fe, CA 92067 (US); BRAMS, Peter, San Diego, CA 92116 (US); HEARD, Cheryl, Encinitas, CA 92024 (US)
(74) Representative: Adams, Harvey Vaughan John
(86) International application number: PCT/US1997/019906
(87) International publication number: WO 1998/019706

(56) References cited:
- WO-A-96/40878
- US-A- 5 434 131
- US-A- 5 521 288
- VAN GOOL S W ET AL: "Synergy between cyclosporin A and a monoclonal antibody to B7 in blocking alloantigen-induced T-cell activation." BLOOD, (1994 JAN 1) 83 (1) 176-83. , XP000973269
- NAKAJIMA A ET AL: "Preferential dependence of autoantibody production in murine lupus on CD86 costimulatory molecule." EUROPEAN JOURNAL OF IMMUNOLOGY, (1995 NOV) 25 (11) 3060-9. , XP000973258
- LENSCHOW D J ET AL: "Inhibition of transplant rejection following treatment with anti- B7 - 2 and anti- B7 - 1 antibodies." TRANSPLANTATION, (1995 NOV 27) 60 (10) 1171-8. , XP000973212
- J. EXP. MED., March 1991, Volume 173, LINSLEY et al., "Binding of the B Cell Activation Antigen B7 to CD28 Costimulates T Cell Proliferation and Interleukin 2 mRNA Accumulation", pages 721-730, XP000601682.
- PROC. NATL. ACAD. SCI. U.S.A., July 1990, Volume 87, LINSLEY et al., "T-Cell Antigen CD28 Mediates Adhesion with B Cells by Interacting with Activation Antigen B7/BB-1", pages 5031-5035, XP002945122.

## Description

### FIELD OF THE INVENTION

The present invention relates to the identification and use of monoclonal antibodies which are specific to B7.1 antigens (CD80). More specifically, the present invention relates to the identification and use of monoclonal antibodies or primatized forms thereof which are capable of inhibiting the binding of human B7.1 antigen to a CD28 receptor and which are not capable of inhibiting the binding of B7.1 to a CTLA-4 receptor. Thus, the invention relates to the identification and use of monoclonal antibodies and primatized forms thereof which recognize specific sites on the B7.1 antigen which are exclusive of CTLA-4 receptor binding.

The invention further relates to monoclonal antibodies or primatized forms thereof which recognize specific sites on the human B7.1 antigen and are capable of inhibiting IL-2 production.

Also, the present invention relates to pharmaceutical compositions containing monoclonal or primatized antibodies specific to human B7.1 and their use as immunosuppressants by modulating the B7:CD28 pathway, e.g., for the treatment of autoimmune disorders, and the prevention of organ rejection.

### BACKGROUND OF THE INVENTION

The clinical interface between immunology, hematology, and oncology has long been appreciated. Many conditions treated by the hematologist or oncologist have either an autoimmune or immunodeficient component to their pathophysiology that has led to the widespread adoption of immunosuppressive medications by hematologists, whereas oncologists have sought immunologic adjuvants that might enhance endogenous immunity to tumors. To date, these interventions have generally consisted of nonspecific modes of immunosuppression and immune stimulation. In addition to the limited efficacy of these interventions, toxicities secondary to their nonspecificity have also limited their overall success. Therefore, alternative strategies have been sought.

Elucidation of the functional role of a rapidly increasing number of cell surface molecules has contributed greatly to the integration of immunology with clinical hematology and oncology. Nearly 200 cell surface antigens have been identified on cells of the immune and hematopoietic systems (Schlossman SF, Boumsell L, Gilks JM, Harlan T, Kishimoto, C Morimoto C, Ritz J., Shaw S, Silverstein RL, Springer TA, Tedder TF, Todd RF:CD antigens (1993), Blood 83:879, 1994). These antigens represent both lineage-restricted and more widely distributed molecules involved in a variety of processes, including cellular recognition, adhesion, induction and maintenance of proliferation, cytokine secretion, effector function, and even cell death. Recognition of the functional attributes of these molecules has fostered novel attempts to manipulate the immune response. Although molecules involved in cellular adhesion and antigen-specific recognition have previously been evaluated as targets of therapeutic immunologic intervention, recent attention has focused on a subgroup of cell surface molecules termed co-stimulatory molecules (Bretscher P: "The two-signal model of lymphocyte activation twenty-one years later." Immunol. Today 13:73, (1992); Jenkins MK, Johnson JG: "Molecules involved in T-cell co-stimulation." Curr Opin Immunol 5:351, (1993); Geppert T, Davis L. Gur H. Wacholtz M. Lipsky P: "Accessory cell signals involved in T-cell activation." Immunol Rev 117:5, (1990); Weaver CT, Unanue ER: "The co-stimulatory function of antigen-presenting cells." Immunol Today 11:49, (1990); Stennam RM, Young JW: "Signals arising from antigen-presenting cells." Curr Opin Immunol 3:361, (1991)). Co-stimulatory molecules do not initiate but rather enable the generation and amplification of antigen-specific T-cell responses and effector function (Bretscher P: "The two-signal model of lymphocyte activation twenty-one years later." Immunol. Today 13:73, (1992); Jenkins MK, Johnson JG: "Molecules involved in T-cell co-stimulation." Curr Opin Immunol 5:351, (1993); Geppert T, Davis L. Gur H. Wacholtz M. Lipsky P: "Accessory cell signals involved in T-cell activation." Immunol Rev 117:5, (1990); Weaver CT, Unanue ER: "The co-stimulatory function of antigen-presenting cells." Immunol Today 11:49, (1990); Stennam RM, Young JW: "Signals arising from antigen-presenting cells." Curr Opin Immunol 3:361, (1991); June CH, Bluestone JA, Linsley PS, Thompson CD: "Role of the CD28 receptor in T-cell activation." Immunol Today 15:321, (1994)).

Recently, one specific co-stimulatory pathway termed B7:CD28 has been studied by different research groups because of its significant role in B and T cell activation (June CH, Bluestone JA, Linsley PS, Thompson CD: "Role of the CD28 receptor in T-cell activation." Immunol Today 15:321, (1994); June CH, Ledbetter JA: "The role of the CD28 receptor during T-cell responses to antigen." Annu Rev Immunol 11:191, (1993); Schwartz RH: "Co-stimulation of T lymphocytes: The role of CD28, CTLA-4, and H7/BB1 in interleukin-2 production and immunotherapy." Cell 71:1065-1068, (1992); Jenkins MK, Taylor PS, Norton SD, Urdahl KB: "CD28 delivers a costimulatory signal involved in antigen-specific IL-2 production by human T cells." Journal of Immunology 147:2461-2466 (1991)). Since this ligand:receptor pathway was discovered four years ago, a large body of evidence has accumulated suggesting that B7:CD28 interactions represent one of the critical junctures in determining immune reactivity versus anergy (June CH, Bluestone JA, Linsley PS, Thompson CD: "Role of the CD28 receptor in T-cell activation." Immunol Today 15:321, (1994); June CH, Ledbetter JA: "The role of the CD28 receptor during T-cell responses to antigen." Annu Rev Immunol 11:191, (1993); Schwartz RH: "Co-stimulation of T lymphocytes: The role of CD28, CTLA-4, and B7/BB1 in interleukin-2 production and immunotherapy." Cell 71:1065-1068, (1992); Cohen J: "Mounting a targeted strike on unwanted immune responses" (news; comment). Science 257:751, (1992); Cohen J: "New protein steals the show as 'co-stimulator' of T cells" (news; comment). Science 262:844, (1993)).

In particular, the role of the human B7 antigens, i.e., human B7.1 (CD80) and B7.2 (CD86), has been reported to play a co-stimulatory role in T-cell activation. See, e.g., Gimmi CD, Freeman, GJ, Gribben JG, Sugita K, Freedman AS, Morimoto C, Nadler LM: "B-cell surface antigen B7 provides a costimulatory signal that induces T cells to proliferate and secrete interleukin 2." Proc. Natl. Acad. Sci. (USA) 88:6575-6579 (1991).

### 1. B7.1 and B7.2 Co-stimulatory Role in T Cell Activation

The elaboration of a successful immune response depends on a series of specific interactions between a T cell and an antigen presenting cell. Although the essential first step in this process depends upon the binding of antigen to the T cell receptor, in the context of the MHC class II molecule (Lane, P.J.L., F.M. McConnell, G.L. Schieven, E.A. Clark, and J.A. Ledbetter, (1990), "The Role of Class II Molecules in Human B Cell Activation." The Journal of Immunology, 144:3684-3692), this interaction alone is not sufficient to induce all the events necessary for a sustained response to a given antigen (Schwartz, R.H. (1990), "A Cell Culture Model for T Lymphocyte Clonal Anergy." Science, 248:1349; Jenkins, M.K. (1992), "The Role of Cell Division in the Induction of Clonal Anergy." Immunology Today, 13:69; Azuma, M., M. Cayabyab, D. Buck, J.H. Phillips, and L.L. Lanier, (1992), "Involvement of CD28 in MHC-unrestricted Cytotoxicity Mediated by a Human Natural Killer Leukemia Cell Line." The Journal of Immunology, 149:1115-1123; Azuma, M., M. Cayabyab, D. Buck, J.H. Phillips, and L.L. Lanier, (1992), "CD28 Interaction with B7 Costimulates Primary Allogeneic Proliferative Responses and Cytotoxicity Mediated by Small Resting T Lymphocytes." J. Exp. Med., 175:353-360); S.D. Norton, L. Zuckerman, K.B. Urdahl, R. Shefner, J. Miller, and M.K. Jenkins, (1992), "The CD28 Ligand, B7, Enhances IL-2 Production by Providing a Costimulatory Signal to T Cells." The Journal of Immunology, 149:1556-1561; R. H. Schwartz, (1992), "Costimulation of T Lymphocytes: The Role of CD28, CTLA-4, and B7/BB1 in Interleukin-2 Production and Immunotherapy." Cell 71:1065-1068).

The involvement of certain other co-stimulatory molecules is necessary (Norton, S.D., L. Zuckerman, K.B. Urdahl, R. Shefner, J. Miller, and M.K. Jenkins, (1992), "The CD28 Ligand, B7, Enhances IL-2 Production by Providing A Costimulatory Signal to T Cells." The Journal of Immunology, 149:1556-1561). "The homodimers CD28 and CTLA-4 expressed on T cells" (June, C.H., J.A. Ledbetter, P.S. Linsley, and C.B. Thompson, (1990), "Role of the CD28 Receptor in T-Cell Activation." Immunology Today, 11:211-216; Linsley, P.S., W. Brady, M. Urnes, L.S. Grosmaire, N.K. Damle, and J.A. Ledbetter, (1991), "CTLA-4 is a Second Receptor for the B Cell Activation Antigen B7." J. Exp. Med., 174:561), together with B7.1 (CD80) and B7.2 (CD86) expressed on antigen presenting cells, are major pairs of co-stimulatory molecules necessary for a sustained immune response (Azuma, M., H. Yssel, J.H. Phillips, H. Spits, and L.L. Lanier, (1993), "Functional Expression of B7/BB1 on Activated T Lymphocytes." J. Exp. Med., 177:845-850; Freeman, G.J., A.S. Freedman, J.M. Segil, G. Lee, J.F. Whitman, and LM. Nadler, (1989), "B7, A New Member of the Ig Superfamily with Unique Expression on Activated and Neoplastic B Cells." The Journal of Immunology, 143:2714-2722; Hathcock, K.S., G. Laslo, H.B. Dickler, J. Bradshaw, P. Linsley, and R.J. Hodes, (1993), "Identification of an Alternative CTLA-4 Ligand Costimulatory for T Cell Activation." Science, 262:905-911; Hart, D.N.J., G.C. Starling, V.L. Calder, and N.S. Fernando, (1993). "B7/B8-1 is a Leucocyte Differentiation Antigen on Human Dendritic Cells Induced by Activation." Immunology, 79:616-620). It can be shown *in vitro* that the absence of these co-stimulatory signals leads to an aborted T cell activation pathway and the development of unresponsiveness to the specific antigen, or anergy. (See, e.g., Harding, F.A., J.G. McArthur, J.A. Gross, D.M. Raulet, and J.P. Allison, (1992), "CD28 Mediated Signalling Co-simulates Murine T Cells and Prevents Induction of Anergy in T Cell Clones." Nature, 356:607-609; Gimmi, C.D., G.J. Freeman, J.G. Gribben, G. Gray, and L.M. Nadler, (1993); "Human T-Cell Clonal Anergy is Induced by Antigen Presentation in the Absence of B7 Costimulation." Proc. Natl. Acad. Sci., 90:6586-6590; Tan, P., C. Anasefti, J.A. Hansen, J. Melrose, M. Brunvand, J. Bradshaw, J.A. Ledbetter, and P.S. Linsley, (1993), "Induction of Alloantigen-specific Hyporesponsiveness in Human T Lymphocytes by Blocking Interaction of CD28 with Its Natural Ligand B7/BB1." J. .Exp. Med., 177:165-173). Achievement of in vivo tolerance constitutes a mechanism for immunosuppression and a viable therapy for organ transplant rejection and for the treatment of autoimmune diseases. This has been achieved in experimental models following the administration of CTLA-4Ig (Lenschow, D.J., Y. Zeng, R.J. Thistlethwaite, A. Montag, W. Brady, M.G. Gibson, P.S. Linsley, and J.A. Bluestone, (1992), "Long-Term Survival of Xenogeneic Pancreatic Islet Grafts Induced by CTLA-4Ig." Science, 257:789-795).

The molecules B7.1 and B7.2 can bind to either CD28 or CTLA-4, although B7.1 binds to CD28 with a Kd of 200 Nm and to CTLA-4 with a 20-fold higher affinity (Linsley, P.S., E.A. Clark, and J.A. Ledbetter, (1990), "T-Cell Antigen CD28 Mediates Adhesion with B Cells by Interacting with Activation Antigen B7/BB-1." Proc. Natl. Acad. Sci., 87:5031-5035; Linsley et al, (1993), "The Role of the CD28 receptor during T cell responses to antigen," Annu. Rev. Immunol., 11:191-192; Linesley et al, (1993), "CD28 Engagement by B7/BB-1 Induces Transient Down-Regulation of CD28 Synthesis and Prolonged Unresponsiveness to CD28 Signaling," The Journal of Immunology, 150:3151-3169). B7.1 is expressed on activated B cells and interferon induced monocytes, but not resting B cells (Freeman, G.J., G.S. Gray, C.D. Gimmi, D.B. Lomarrd, L-J. Zhou, M. White, J.D. Fingeroth, J.G. Gribben, and LM. Nadler, (1991). "Structure, Expression and T Cell Costimulatory Activity of the Murine Homologue of the Human B Lymphocyte Activation Antigen B7," J. Exp. Med., 174:625-631). B7.2, on the other hand, is constitutively expressed at very low levels on resting monocytes, dendritic cells and B cells, and its expression is enhanced on activated T cells, NK cells and B lymphocytes (Azuma, M. D. Ito, H. Yagita, K. Okumura, J.H. Phillips, L.L. Lanier, and C. Somoza, 1993, "B70 Antigen is a Second Ligand for CTLA-4 and CD28," Nature, 366:76-79). Although B7.1 and B7.2 can be expressed on the same cell type, their expression on B cells occurs with different kinetics (Lenschow, D.J., G.H. Su, L.A. Zuckerman, N. Nabavi, C.L. Jellis, G.S. Gray, J. Miller, and J.A. Bluestone, (1993), "Expression and Functional Significance of an Additional Ligand for CTLA-4," Proc. Natl. Acad. Sci., USA, 90:11054-11058; Boussiotis, V.A., G.J. Freeman, J.G. Gribben, J. Daley, G. Gray, and L.M. Nadler, (1993), "Activated Human B Lymphocytes Express Three CTLA-4 Counter-receptors that Co-stimulate T-Cell Activation." Proc. Natl. Acad. Sci., USA, 90:11059-11063). Further analysis at the RNA level has demonstrated that B7.2 mRNA is constitutively expressed, whereas B7.1 MRNA is detected 4 hours after activation and initial low levels of B7.1 protein are not detectable until 24 hours after stimulation (Boussiotis, V.A., G.J. Freeman, J.G. Gribben, J. Daley, G. Gray, and L.M. Nadler, (1993), "Activated Human B Lymphocytes Express Three CTLA-4 Counter-receptors that Co-stimulate T-Cell Activation," Proc. Natl. Acad. Sci., USA, 90:11059-11063). CTLA-4/CD28 counter receptors, therefore, may be expressed at various times after B Cell activation.

More recently, it has been suggested that the second T cell associated co-receptor CTLA-4 apparently functions as a negative modulator to override and prevent a runaway immune system (Krummel M, Allison J: "CD28 and CTLA-4 have opposing effects on the response of T cells to stimulation." J. Exp. Med. 182:459-466 (1995)). The CTLA-4 receptor plays a critical role in down regulating the immune response, as evidenced in CTLA-4 knockout mice. Knockout mice born without the ability to express the CTLA-4 gene die within 3-4 weeks of severe lymphoproliferative disorder (Tivol EA, Borriello G, Schweitzer AN, Lynch WP, Bluestone JA, Sharpe AH: "Loss of CTLA-4 leads to massive lymphoproliferation and fatal multiorgan tissue destruction, revealing a critical negative regulatory role of CTLA-4." Immunity 3:541-547 (1995)). CTLA-4 is thought to function through signaling mechanisms linked to induction of apoptosis (Gribben JG, Freeman GJ, Boussiotis VA, Rennert P, Jellis CL, Greenfield E, Barber M, Restivo Jr. VA, Ke X, Gray GS, Nadler LM: "CTLA-4 mediates antigen specific apoptosis of human T cells." Proc. Natl. Acad. Sci. (USA) 92:811-815 (1995)), triggered through as yet undefined ligand binding to specific cites on the receptor. It has been shown in vitro that the blocking of the B7.1/B7.2 dependent co-stimulatory signals in various ways leads to an aborted T cell activating pathway and the development of unresponsiveness to the specific antigen (Lederman S, Chess L, Yellin MJ: "Murine monoclonal antibody (5c8) recognizes a human glycoprotein on the surface of T-lymphocytes, compositions containing same." U.S. Patent No. 5,474.771 (December 12, 1995); Linsley PS, Ledbetter JA, Damle NK, Brady W: "Chimeric CTLA4 receptor and methods for its use." U.S. Patent No. 5,434,131 (July 18, 1995); Harding, 1992; Gimmi CD, Freeman GJ, Bribben JG, Gray G, Nadler LM: "Human T-cell clonal anergy is induced by antigen presentation in the absence of B7 costimulation." Proc. Natl. Acad. Sci. (USA) 90:6586-6590 (1993); Tan P, Anasetti C, Hansen JA, Melrose J, Brunvand M, Bradshaw J, Ledbetter JA, Linsley PS: "Induction of alloantigen-specific hyporesponsiveness in human T lymphocytes by blocking interaction of CD28 with its natural ligand B7/BB1." J. Exp. Med. 177:165-173 (1993)). Achievement of in vivo tolerance, anergy, or depleting of antigen-specific T cells would constitute a mechanism for immunosuppression and a viable therapy for organ transplant rejection or plausible treatment for autoimmune diseases.

The differential temporal expression of B7.1 and B7.2 suggests that the interaction of these two molecules with CTLA-4 and/or CD28 deliver distinct but related signals to the T cell (LaSalle, J.M., P.J. Tolentino, G.J. Freeman, L.M. Nadler, and D.A. Hafler, (1992), "CD28 and T Cell Antigen Receptor Signal Transduction Coordinately Regulate Interleukin 2 Gene Expression In Response to Superantigen Stimulation," J. Exp. Med., 176:177-186; Vandenberghe, P., G.J. Freeman, L.M. Nadler, M.C. Fletcher, M. Kamoun, L.A. Turka, J.A. Ledbetter, C.B. Thompson, and C.H. June, (1992), "Antibody and B7/BB1-mediated Ligation of the CD28 Receptor Induces Tyrosine Phosphorylation in Human T Cells," The Journal of Experimental Medicine, 175:951-960). The exact signaling functions of CTLA-4 and CD28 on the T cell are currently unknown (Janeway, C.A., Jr. and K. Bottomly, (1994), "Signals and Signs for Lymphocyte Responses," Cell, 76.275285). However, it is possible that one set of receptors could provide the initial stimulus for T cell activation and the second, a sustained signal to allow further elaboration of the pathway and clonal expansion to take place (Linsley, P.S., J.L. Greene, P. Tan, J. Bradshaw, J.A. Ledbetter, C. Anasetti, and N.K. Damle, (1992), "Coexpression and Functional Cooperation of CTLA-4 and CD28 on Activated T Lymphocytes," J. Exp. Med., 176:1595-1604). The current data supports the two-signal hypothesis proposed by Jenkins and Schwartz (Schwartz, R.H., (1990), "A Cell Culture Model for T Lymphocyte Clonal Anergy," Science, 248:1349; Jenkins, M.K., (1992), "The Role of Cell Division in the Induction of Clonal Anergy," Immunology Today, 13:69) that both a TCR and co-stimulatory signal are necessary for T cell expansion, lymphokine secretion and the full development of effector function (Greenan, V. and G. Kroemer, (1993), "Multiple Ways to Cellular Immune Tolerance," Immunology Today, 14:573). The failure to deliver the second signal results in the inability of T cells to secrete IL-2 and renders the cell unresponsive to antigen.

Structurally, both B7.1 and B7.2 contain extracellular immunoglobulin superfamily V and C-like domains, a hydrophobic transmembrane region and a cytoplasmic tail (Freeman, G.J., J.G. Gribben, V.A. Boussiotis, J.W. Ng, V. Restivo, Jr., L.A. Lombard, G.S. Gray, and L.M. Nadler, (1993), "Cloning of B7.2: A CTLA-4 Counter-receptor that Co-stimulates Human T Cell Proliferation," Science, 262:909). Both B7.1 and B7.2 are heavily glycosylated. B7.1 is a 44-54kD glycoprotein comprised of a 223 amino acid extracellular domain, a 23 amino acid transmembrane domain, and a 61 amino acid cytoplasmic tail. B7.1 contains 3 potential protein kinase phosphorylation sites. (Azuma, M., H. Yssel, J.H. Phillips, H. Spits, and L.L. Lanier, (1993), "Functional Expression of B7/BB1 on Activated T Lymphocytes," J. Exp. Med., 177:845-850). B7.2 is a 306 amino acid membrane glycoprotein. It consists of a 220 amino acid extracellular region, a 23 amino acid hydrophobic transmembrane domain and a 60 amino acid cytoplasmic tail (Freeman, G.J., A.S. Freedman, J.M. Segil, G. Lee, J.F. Whitman, and LM. Nadler, (1989), "B7, A New Member of the Ig Superfamily with Unique Expression on Activated and Neoplastic B Cells," The Journal of Immunology, 143:2714-2722). Although both B7.1 and B7.2 genes are localized in the same chromosomal region (Freeman, G.J., D.B. Lombard, C.D. Gimmi, S.A. Brod, L Lee, J.C. Laning, D.A. Hafler, M.E. Dorf, G.S. Gray, H. Reiser, C.H. June, C.B. Thompson, and L.M. Nadler, (1992), "CTLA-4 and CD28 MRNA are Coexpressed in Most T Cells After Activation," The Journal of Immunology, 149:3795-3801; Schwartz, R.H., (1992), "Costimulation of T Lymphocytes: The Role of CD28, CTLA-4, and B7/BB1" in Selvakumar, A., B.K. Mohanraj, R.L. Eddy, T.B. Shows, P.C. White, C. Perrin, and B. Dupont, (1992), "Genomic Organization and Chromosomal Location of the Human Gene Encoding the B-Lymphocyte Activation Antigen B7," Immunogenetics, 36:175-181), these antigens do not share a high level of homology. The overall homology between B7.1 and B7.2 is 26% and between murine B7.1 and human B7.1 is 27% (Azuma, M., H. Yssel, J.H. Phillips, H. Spits, and L.L. Lanier, (1993), "Functional Expression of B7/BB1 on Activated T Lymphocytes," J. Exp. Med., 177:845-850; Freeman, G.J., A.S. Freedman, J.M. Segil, G. Lee, J.F. Whitman, and LM. Nadler, (1989), "B7, A New Member of the Ig Superfamily with Unique Expression on Activated and Neoplastic B Cells," The Journal of Immunology, 143:2714-2722). Although alignment of human B7.1 human B7.2 and murine B7.1 sequences shows few stretches of lengthy homology, it is known that all three molecules bind to human CTLA-4 and CD28. Thus, there is most likely a common, or closely homologous region shared by the three molecules that may be either contiguous or conformational. This region may constitute the binding site of the B7.1 and B7.2 molecules to their counter-receptors. Antibodies raised against these epitopes could potentially inhibit the interaction of B7 with its counter-receptor on the T cell. Furthermore, antibodies that cross-reacted with this region on both B7.1 and B7.2 molecules would potentially have practical advantages over antibodies directed against B7.1 or B7.2 separately.

### 2. Blockade of the B7/CD28 Interaction

Blocking of the B7/CD28 interaction offers the possibility of inducing specific immunosuppression, with potential for generating long lasting antigen-specific therapeutic effects. Antibodies or agents that temporarily prevent this interaction may be useful, specific and safe clinical immunosuppressive agents, with potential for generating long term antigen-specific therapeutic effects.

Antibodies to either B7.1 or B7.2 have been shown to block T cell activation, as measured by the inhibition of IL-2 production in vitro (DeBoer, M., P. Parren, J. Dove, F. Ossendorp, G. van der Horst, and J. Reeder, (1992), "Functional Characterization of a Novel Anti-B7 Monoclonal Antibody," Eur. Journal of Immunology, 22:3071-3075; Azuma, M., H. Yssel, J.H. Phillips, H. Spits, and L.L. Lanier, (1993), "Functional Expression of B7/BB1 on Activated T Lymphocytes," J. Exp. Med., 177:845-850). However, different antibodies have been shown to vary in their immunosuppressive potency, which may reflect either their affinity or epitope specificity. A possible explanation for this may reside in the ability of some antibodies to block only the binding of B7 to CD28, while promoting apoptosis or some other form of negative signaling through the CTLA-4 receptor in activated T cells. Some antibodies to B7.1 or B7.2 may, in fact, hinder the activity of CTLA-4 by cross-reacting with the CTLA-4 binding domain. CTLA-4Ig fusion protein and anti-CD28 Fabs were shown to have similar effects on the down regulation of IL-2 production.

*In vivo* administration of a soluble CTLA-4Ig fusion protein has been shown to suppress T cell dependent antibody responses in mice (Linsley, P.S., J.L. Greene, P. Tan, J. Bradshaw, J.A. Ledbetter, C. Anasetti, and N.K. Damle, (1992), "Coexpression and Functional Cooperation of CTLA-4 and CD28 on Activated T Lymphocytes," J. Exp. Med., 176:1595-1604; Lin, H., S.F. Builing, P.S. Linsley, R.O. Wei, C.D. Thompson, and L.A. Turka, (1993), "Long-term Acceptance of Major Histocompatibility Complex Mismatched Cardiac Allografts Induced by CTLA-4-Ig Plus Donor Specific Transfusion," J. Exp. Med., 178:1801) and, furthermore, larger doses were also able to suppress responses to a second immunization, demonstrating the feasibility of this approach for the treatment of antibody mediated autoimmune disease. In addition, CTLA-4Ig was able to prevent pancreatic islet cell rejection in mice by directly inhibiting the interaction of T cells and B7.1/B7.2 antigen presenting cells (Lenschow, D.J., G.H. Su, L.A. Zuckerman, N. Nabavi, C.L. Jellis, G.S. Gray, J. Miller, and J.A. Bluestone, (1993), "Expression and Functional Significance of an Additional Ligand for CTLA-4," Proc. Natl. Acad. Sci., USA, 90:11054-11058). In this case, long term donor specific tolerance was achieved.

### 3. Recombinant Phage Display Technology for Antibody Selection

To date, no monoclonal antibodies which crossreact with both B7.1 and B7.2 have been reported. Furthermore, no monoclonal antibodies which are specific to B7.1 or B7.2 and which also recognize specific sites on the antigens which are restricted to co-activation receptor CD28 binding have been reported. Or alternatively, no monoclonal antibodies which are specific to B7.1 or B7.2 and which recognize specific sites on the antigens which are exclusive of CTLA-4 receptor binding have been reported. As discussed supra, such antibodies would potentially be highly desirable as immunosuppressants.

Phage display technology is beginning to replace traditional methods for isolating antibodies generated during the immune response, because a much greater percentage of the immune repertoire can be assessed than is possible using traditional methods. This is in part due to PEG fusion inefficiency, chromosomal instability, and the large amount of tissue culture and screening associated with heterohybridoma production. Phage display technology, by contrast, relies on molecular techniques for potentially capturing the entire repertoire of immunoglobulin genes associated with the response to a given antigen.

This technique is described by Barbas et al, Proc. Natl. Acad. Sci., USA, 88, 7978-7982, (1991).
Essentially, immunoglobulin heavy chain genes are PCR amplified and cloned into a vector containing the gene encoding the minor coat protein of the filamentous phage M13 in such a way that a heavy chain fusion protein is created. The heavy chain fusion protein is incorporated into the M13 phage particle together with the light chain genes as it assembles. Each recombinant phage contains, within its genome, the genes for a different antibody Fab molecule which it displays on its surface. Within these libraries, in excess of 10⁶ different antibodies can be cloned and displayed. The phage library is panned on antigen coated microliter wells, non-specific phage are washed off, and antigen binding phage are eluted. The genome from the antigen-specific clones is isolated and the gene III is excised, so that antibody can be expressed in soluble Fab form for further characterization. Once a single Fab is selected as a potential therapeutic candidate, it may easily be converted to a whole antibody. A previously described expression system for converting Fab sequences to whole antibodies is IDEC's mammalian expression vector NEOSPLA. This vector contains either human gamma 1 or gamma 4 constant region genes. CHO cells are transfected with the NEOSPLA vectors and after amplification this vector system has been reported to provide very high expression levels (> 30 pg/cell/day) can be achieved.

### 4. Primatized Antibodies

Another highly efficient means for generating recombinant antibodies is disclosed by Newman, (1992), Biotechnology, 10, 1455-1460. More particularly, this technique results in the generation of primatized antibodies which contain monkey variable domains and human constant sequences.

Moreover, this technique is also described in commonly assigned U.S. Application No. 08/379,072, corresponding to PCT/US92/06194 which was published as WO 93/02108;

This technique modifies antibodies such that they are not antigenically rejected upon administration in humans. This technique relies on immunization of cynomolgus monkeys with human antigens or receptors. This technique was developed to create high affinity monoclonal antibodies directed to human cell surface antigens.

Identification of macaque antibodies to human B7.1 and B7.2 by screening of phage display libraries or monkey heterohybridomas obtained using B lymphocytes from B7.1 and/or B7.2 immunized monkeys is also described in commonly assigned U.S. Application No. 08/487, 550, filed June 7, 1995, corresponding to PCT/US96/10053 which was published as WO 96/40878. More specifically, 08/487,550 provides four monoclonal antibodies 7B6, 16C10, 7C10 and 20C9 which inhibit the B7:CD28 pathway and thereby function as effective immunosuppressants. WO 96/40878 falls under A54(3) EPC.

Antibodies generated in the manner described by these co-assigned applications have previously been reported to display human effector function, have reduced immunogenicity, and long serum half-life. The technology relies on the fact that despite the fact that cynomolgus monkeys are phylogenetically similar to humans, they still recognize many human proteins as foreign and therefore mount an immune response. Moreover, because the cynomolgus monkeys are phylogenetically close to humans, the antibodies generated in these monkeys have been discovered to have a high degree of amino acid homology to those produced in humans. Indeed, after sequencing macaque immunoglobulin light and heavy chain variable region genes, it was found that the sequence of each gene family was 85-98% homologous to its human counterpart (Newman et al, (1992), Id.). The first antibody generated in this way, an anti-CD4 antibody, was 91-92% homologous to the consensus sequence of human immunoglobulin framework regions. Newman et al, Biotechnology, 10:1458-1460, (1992).

Monoclonal antibodies specific to the human B7 antigen have been previously described in the literature. For example, Weyl et al, Hum. Immunol., 31(4), 271-276, (1991) describe epitope mapping of human monoclonal antibodies against HLA-B-27 using natural and mutated antigenic variants. Also, Toubert et al, Clin. Exp. Immunol., 82(1), 16-20, (1990) describe epitope mapping of an HLA-B27 monoclonal antibody that also reacts with a 35-KD bacterial outer membrane protein. Also, Valle et al, Immunol., 69(4), 531-535, (1990) describe a monoclonal antibody of the IgG1 subclass which recognizes the B7 antigen expressed in activated B cells and HTLV-1-transformed T cells. Further, Toubert et al, J. Immunol., 141(7), 2503-9, (1988) describe epitope mapping of HLA-B27 and HLA-B7 antigens using intradomain recombinants constructed by making hybrid genes between these two alleles in E. coli.

Van Gool et al. (Blood, 1994, 83: 176-183) shows that a monoclonal anti-CD80 antibody that efficiently blocks the interaction of CD80 with CD28 partially inhibits T cell proliferation and IL-2 production but does not affect generation of cytotoxic T lymphocytes in mixed lymphocyte reactions *in vitro*.

Nakajima et al. (Eur. J. Immunol., 1995, 25: 3060-3069) shows that administration of a combination of monoclonal anti-CD80 and anti-CD86 antibodies to lupus-prone mice before the onset of lupus completely prevents autoantibody production and nephritis and prolonged survival in the mice. The effect was not observed when monoclonal anti-CD80 antibodies were administered alone.

Lenschow et al. (Transplantation, 1995, 60: 1171-1178) uses monoclonal anti-CD80 and anti-CD86 antibodies to study the co-stimulatory roles of CD80 and CD86 in T cell activation in a murine allogeneic pancreatic islet transplant system, and shows that administration of a combination of monoclonal anti-CD80 and anti-CD86 antibodies suppresses T cell proliferative responses and prolongs allograft survival more effectively than either antibody alone.

high expression of B7 antigen has been correlated to autoimmune diseases by some researchers. For examples, Ionesco-Tirgoviste et al, Med. Interre, 24(1), 11-17, (1986) report increased B7 antigen expression in type 1 insulin-dependent diabetes. Also, the involvement of B7 antigen expression on dermal dendritic cells obtained from psoriasis patients has been reported. (Nestle et al, J. Clin. Invest. , 94(1), 202-209, (1994)).

Further, the inhibition of anti-HLA-B7 alloreactive CTL using affinity-purified soluble-HLA-H7 has been reported in the literature. (Zavazava et al, Transplantation, 51(4), 838-42, (1991)). Further, the use of B7 receptor soluble ligand, CTLA-4-Ig to block B7 activity (See, e.g., Lenschow et al, Science, 257, 789, 7955 (1992)) in animal models and a B7.1-Ig fusion protein capable of inhibiting B7 has been reported.

Evidence is provided in this disclosure for the identification of monoclonal antibodies which recognize specific sites on the B7.1 antigen which are restricted to CD28 receptor binding. Furthermore, evidence is presented herein for the identification of antibodies which recognize sites on the B7.1 antigen which are exclusive of CTLA-4 receptor binding. Thus, evidence is presented herein to support the existence of unique antigen binding sites on the human B7.1 (CD80) co-stimulatory antigen. The sites claimed are identified bey anti-B7.1 PRIMATIZED^{®} antibodies and evidence is presented which confirms binding to a site of interaction on the B7.1 antigen which is restricted to binding with the co-activation receptor CD28.

### SUMMARY AND OBJECTS OF THE INVENTION

An object of the invention is to identify novel antibodies which are specific to human B7.1 antigen. More specifically, it is an object of the invention to identify antibodies which are specific to human B7.1 antigen and which are also capable of inhibiting the binding of B7.1 to a CD28 receptor. It is also an object of this invention to identify antibodies which are specific to human B7.1 antigen and which are not capable of inhibiting the binding of B7.1 to a CTLA-4 receptor. Thus, an object of this invention is to identify antigens which recognize specific sites on the B7.1 antigen, wherein the recognized sites are restricted to CD28 receptor binding and which are exclusive of CTLA-4 receptor binding.

It is a further object of the invention to identify antibodies which are specific to human B7.1 antigen and which fail to recognize human B7.2 antigen.

It is another object of the invention to identify monoclonal antibodies and primatized forms thereof which recognize specific sites on the human B7.1 antigen and which inhibit IL-2 production and T cell proliferation and which function as effective immunosuppressants. More specifically, it is an object of this invention to identify antibodies which are specific to B7.1 and which are capable of inhibiting IL-2 production.

It is another object of the invention to provide monoclonal antibodies and primatized forms thereof which inhibit antigen driven responses in donor spleen cell cultures, e.g., antigen specific IgG responses, IL-2 production and cell proliferation.

It is another specific object of the invention to identify particular monoclonal antibodies specific to human B7.1 antigen and primatized forms thereof having advantageous properties, i.e., affinity, immunosuppressive activity, which are useful as therapeutics. More specifically, these antibodies and privatized forms thereof are to be used, e.g., as immunosuppressants, i.e., to block antigen driven immune responses, to treat autoimmune diseases such as psoriasis, rheumatoid arthritis, systemic erythematosus (SLE), type 1 diabetes mellitus, idiopathic thrombocytopenia purpura (ITP), allergy, inflammatory bile disease, and to prevent organ rejection.

It is another object of the invention to provide pharmaceutical compositions containing one or more monoclonal antibodies specific to human B7.1 antigen or primatized forms thereof, and a pharmaceutically acceptable carrier or excipient. These compositions will be used, e.g., as immunosuppressants to treat autoimmune diseases, e.g., idiopathic thrombocytopenia purpura (ITP) and systemic lupus erythematosus (SLE), to block antigen driven immune responses, and to prevent organ rejection in transplant recipients.

It is another object of the invention to provide for medicaments that can be used in novel methods of therapy by administration of therapeutically effective amounts of one or more or primatized monoclonal antibodies which specifically bind to human B7.1 antigen. Such therapeutic methods are useful for treatment of diseases treatable by inhibition of the B7:CD28 pathway, e.g., autoimmune diseases such as idiopathic thrombocytopenia purpura (ITP), systemic lupus erythematosus (SLE), type 1 diabetes mellitus, psoriasis, rheumatoid arthritis, multiple sclerosis, aplastic anemia, as well as for preventing rejection in transplantation subjects.

This disclosure also describes transfectants, e.g., CHO cells, which express at least the variable heavy and light domains of monoclonal antibodies specific to the human B7.1 antigen.

### Definitions

The following terms are defined so that the invention may be more clearly understood.
Depleting antibody - an antibody which kills activated B cells or other antigen presenting cells.
Non-depleting antibody - an antibody which blocks the co-stimulatory action of B7 and T cell activating ligands CD28 and CTLA-4, Thus, it energizes but does not eliminate the antigen presenting cell.
Primatized antibody - a recombinant antibody which has been engineered to contain the variable heavy and light domains of a monkey antibody, in particular, a cynomolgus monkey antibody, and which contains human constant domain sequences, preferably the human immunoglobulin gamma 1 or gamma 4 constant domain (or PE variant). The preparation of such antibodies is described in Newman et al, (1992), "Primatization of Recombinant Antibodies for Immunotherapy of Human Diseases : A Macaque/Human Chimeric Antibody Against Human CDH, Biotechnology, 10:3.458-1460; also in commonly assigned 08/379,072, corresponding to PCT/US92/06194 which was published as WO93/02108. These antibodies have been reported to exhibit a high degree of homology to human antibodies, i.e., 85-98%, display human effector functions, have reduced immunogenicity, and may exhibit high affinity to human antigens,
B7 antigens - B7 antigens in this application include, e.g., human B7, B7.1 and B7.2 antigens. These antigens bind to CD28 and/or CTLA-4. These antigens have a co-stimulatory role in T cell activation. Also, these B7 antigens all contain extracellular immunoglobulin superfamily V and C-like domains, a hydrophobic transmembrane region and a cytoplasmic tail. (See, Freeman et al, Science, 262:909, (1993)), and are heavily glycosylated.
Anti-B7 antibodies - Antibodies, preferably monkey monoclonal antibodies or primatized forms thereof, which specifically bind human B7 antigens, e.g., human B7.1 and/or B7.2 antigen with a sufficient affinity to block the B7:CD28 interaction and thereby induce immunosuppression.

### BRIEF DESCRIPTION OF THE FIGURES

Figure 1 depicts the pMS vector used to screen recombinant immunoglobulin libraries produced against B7 displayed on the surface of filamentous phage which contains primers based on macaque immunoglobulin sequences.
Figure 2 depicts the NEOSPLA expression vector used to express the subject primatized antibodies specific to human B7.1 antigen.
Figure 3a depicts the amino acid and nucleic acid sequence of a primatized form of the light chain of 7C10.
Figure 3b depicts the amino acid and nucleic acid sequence of a primatized form of the heavy chain of 7C10.
Figure 4a depicts the amino acid and nucleic acid sequence of a primatized form of the light chain of 7B6.
Figure 4b depicts the amino acid and nucleic acid sequence of a primatized form of the heavy chain of 7B6.
Figure 5a depicts the amino acid and nucleic acid sequence of a primatized light chain 16C10.
Figure 5b depicts the amino acid and nucleic acid sequence of a primatized heavy chain 16C10.
Figure 6 depicts the inability of P16C10 to block CTLA-4Ig-Biotin binding to B7.1 transfected CHO cells.
Figure 7 depicts the inability of CTLA-4Ig to block P16C10-Biotin binding to B7.1 transfected CHO cells.
Figure 8 depicts that BB-1 completely blocks binding of CTLA-4Ig-Biotin to B7.1 transfected CHO cells and further depicts the inability of BB-1 to significantly affect P16C10-Biotin binding to B7.1 transfected CHO cells.
Figure 9 depicts that CTLA-4Ig-Biotin is effectively blocked by all B7.1 inhibitors except P16C10.
Figure 10 depicts the ability of P16C10 to block binding of the CD28/B7-1Ig interaction. Data shown are averages of values obtained from four separate experiments.

### DETAILED DESCRIPTION OF THE INVENTION

As described above, the present invention relates to the identification of monoclonal antibodies or primatized forms thereof which are specific to human B7.1 antigen and which are capable of inhibiting the binding of B7.1 to a CD28 receptor and which are not capable of inhibiting the binding of B7.1 to a CTLA-4 receptor. Blocking of the primary activation site between CD28 and B7.1 (CD80) with the identified antibodies while allowing the combined antagonistic effect on positive co-stimulation with an agnostic effect on negative signaling will be a useful therapeutic approach for intervening in relapsed forms of autoimmune disease. The functional activity of the identified antibodies is defined by blocking the production of the T cell stimulatory cytokine IL-2. Identified antibodies have demonstrated the ability to block the production of IL-2 in excess of 50%, in spite of the existence of a second actuating ligand B7.2, suggesting an alternate mechanism of action exists which is not typical of the observed effects of other anti-B7.1 antibodies defined in the literature.

Manufacture of novel monkey monoclonal antibodies which specifically bind human B7-1 and/or human B7.2 antigen, as well as primatized antibodies derived therefrom is described in co-pending U.S. Application Serial No. 08/487,550, corresponding to PCT/US96/10053 which was published as WO96/40878, and as set forth herein. These antibodies possess high affinity to human B7.1 and/or B7.2 and therefore may be used as immunosuppressants which inhibit the B7:CD86 pathway. WO 96/40878 falls under A54(3) EPC.

Preparation of monkey monoclonal antibodies will preferably be effected by screening of phage display libraries, or by preparation of monkey heterohybridomas using B lymphocytes obtained from B7 (e.g., human B7.1 and/or B7.2) immunized monkeys.

As noted, the first method for generating anti-B7 antibodies involves recombinant phage display technology. This technique is generally described *supra*.

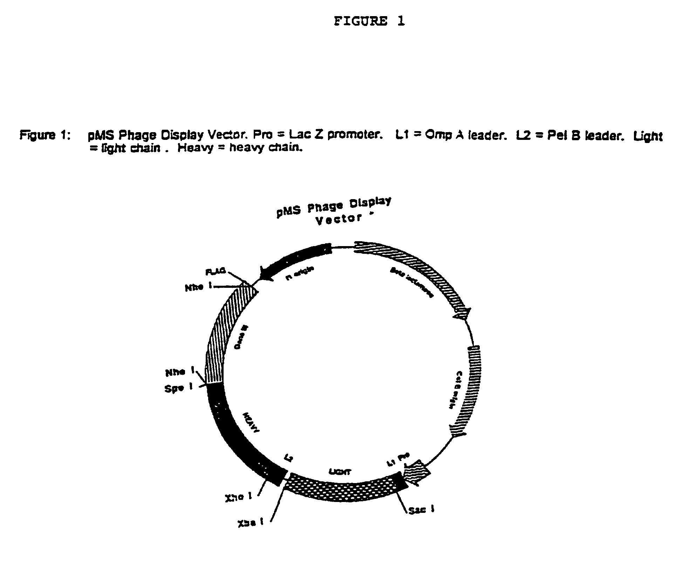

Essentially, this will comprise synthesis of Recombinant immunoglobulin libraries against B7 antigen displayed on the surface of filamentous phage and selection of phage which secrete antibodies having high affinity to B7.1 and/or B7.2 antigen. As noted supra, preferably antibodies will be selected which bind to both human B7.1 and B7.2. To effect such methodology, the present inventors have created a unique library for monkey libraries which reduces the possibility of recombination and improves stability. This vector, PMS, is described in detail infra, and is shown in Figure 1.

Essentially, to adopt phage display for use with macaque libraries, this vector contains specific primers for PCR amplifying monkey immunoglobulin genes. These primers are based on macaque sequences obtained while developing the primatized technology and databases containing human sequences.

Suitable primers are disclosed in commonly assigned 08/379,072, corresponding to PCT/US02/06194 which was published as WO93/02108.

The second method involves the immunization of monkeys, i.e., macaques, against human B7 antigen, preferably against human B7.1 and B7.2 antigen. The inherent advantage of macaques for generation of monoclonal antibodies is discussed *supra*. In particular, such monkeys, i.e., cynomolgus monkeys, may be immunized against human antigens or receptors. Moreover, the resultant antibodies may be used to make primatized antibodies according to the methodology of Newman et al, Biotechnology, 10, 1455-1460, (1992) and Newman et al, commonly assigned U.S. Serial No. 08/379,072, filed January 25, 1995, corresponding to PCT/US02/06194 which was published as WO93/02108.

The significant advantage of antibodies obtained from cynomolgus monkeys is that these monkeys recognize many human proteins as foreign and thereby provide for the formation of antibodies, some with high affinity to desired human antigens, e.g., human surface protein and cell receptors. Moreover, because they are phylogenetically close to humans, the resultant antibodies exhibit a high degree of amino acid homology to those produced in humans. As noted above, after sequencing macaques immunoglobulin light and heavy variable region genes, it was found that the sequence of each gene family was 85-88% homologous to its human counterpart (Newman et al, (1992), Id.).

Essentially, cynomolgus macaque monkeys are administered human B7 antigen, e.g., human B7.1 and/or human B7.2 antigen, B cells are isolated therefrom, e.g., lymph node biopsies are taken from the animals, and B lymphocytes are then fused with KH6/B5 (mouse x human) heteromyeloma cells using polyethylene glycol (PEG). Heterohybridomas secreting antibodies which bind human B7 antigen, e.g., human B7.1 and/or human B7.2 antigen, are then identified.

Antibodies which bind to both B7.1 and B7.2 are desirable because such antibodies potentially may be used to inhibit the interaction of B7.1 and B7.2, as well as B7 with their counter-receptors, i.e., human CTLA-4 and CD28. Antibodies against these epitopes may inhibit the interaction of both human B7.1 and human B7.2 with their counter receptors on the T cell. This may potentially provide synergistic effects.

However, antibodies which bind to only one of human B7 antigen, B7.1 antigen or B7.2 antigen, are also highly desirable because of the co-involvement of these molecules in T cell activation, clonal expansion lymphokine (IL-2) secretion, and responsiveness to antigen. Given that both human B7.1 and B7.2 bind to human CTLA-4 and CD28, it is probable that there is at least one common or homologous region (perhaps a shared conformational epitope or epitopes) to which macaque antibodies may potentially be raised.

Animals can be primed to produce a particular antibody. Animals which are useful for such a process include, but are not limited to, the following: mice, rats, guinea pigs, hamsters, monkeys, pigs, goats and rabbits.

A preferred means of generating human antibodies using SCID mice is disclosed in commonly-owned, co-pending U.S. Patent application Serial No. 08/488,376.

The present inventors elected to immunize macaques against human B7.1 antigen using recombinant soluble B7.1 antigen produced in CHO cells and purified by affinity chromatography using a L307.4-sepharose affinity column. However, the particular source of human B7 antigen, human B7.1 antigen or human B7.2 antigen is not critical, provided that it is of sufficient purity to result in a specific antibody response to the particular administered B7 antigen and potentially to other B7 antigens.

The human B7 antigen, human B7.1 antigen (also called CD80) and human B7.2 antigen (also called CD86) genes have been cloned, and sequenced, and therefore may readily be manufactured by recombinant methods.

Preferably, the administered human B7 antigen, human B7.1 antigen and/or human B7.2 antigen will be administered in soluble form, e.g., by expression of a B7, B7.1 or B7.2 gene which has its transmembrane and cytoplasmic domains removed, thereby leaving only the extracellular portion, i.e., the extracellular superfamily V and C-like domains. (See, e.g., Grumet et al, Hum. Immunol., 40(3), p. 228-234, 1994, which teaches expression of a soluble form of human B7, which is incorporated by reference in its entirety herein).

The macaques will be immunized with the B7, B7.1 and/or B7.2 antigen, preferably a soluble form thereof, under conditions which result in the production of antibodies specific thereto. Preferably, the soluble human B7, B7.1 or B7.2 antigen will be administered in combination with an adjuvant, e.g., Complete Freund's Adjuvant (CFA), Alum, Saponin, or other known adjuvants, as well as combinations thereof. In general, this will require repeated immunization, e.g., by repeated injection, over several months. For example, administration of soluble B7.1 antigen was effected in adjuvant, with booster immunizations, over a 3 to 4 month period, with resultant production of serum containing antibodies which bound human B7.1 antigen.

After immunization B cells are collected, e.g., by lymph node biopsies taken from the immunized animals and B lymphocytes fused with KH6/B5 (mouse x human) heteromyeloma cells using polyethylene glycol. Methods for preparation of such heteromyelomas are known and may be found in U.S. Serial No. 08/379,072 by Newman et al, filed on January 25, 1995, corresponding to PCT/US92/06194 which was published as WO93/02108.

Heterohybridomas which secrete antibodies which bind human B7, B7.1 and/or B7.2 are then identified. This may be effected by known techniques. For example, this may be determined by ELISA or radioimmunoassay using enzyme or radionucleotide labelled human B7, B7.1 and/or B7.2 antigen.

Cell lines which secrete antibodies having the desired specificity to human B7, B7.1. and/or B7.2 antigen are then subcloned to monoclonality.

The inventors screened purified antibodies for their ability to bind to soluble B7.1 antigen coated plates in an ELISA assay, antigen positive B cells, and CHO transfectomas which express human B7.1 antigen on their cell surface. In addition, the antibodies were screened for their ability to block B cell/T cell interactions as measured by IL-2 production and tritiated thymidine uptake in a mixed lymphocyte reaction (MLR), with B7 binding being detected using ¹²⁵I-radiolabeled soluble B7.1 (SB7.1).

Also, affinity purified antibodies from macaques were tested for their reactivity against CHO transfectants which expressed B7.1/Ig fusion proteins, and against CHO cells which produced human B7.2 antigen. These results indicated that the B7.1 immune sera bound to the B7.2 transfectomas. Binding of antibodies to B7.2 antigen may be confirmed using soluble B7.2-Ig reagents. As discussed in the examples, this may be effected by producing and purifying B7.2-Ig from CHO transfectomas in sufficient quantities to prepare a B7.2-Ig-sepharose affinity column. Those antibodies which cross-react with B7.2 will bind the B7.2-Ig-sepharose column.

Cell lines which express antibodies which specifically bind to humane B7 antigen, B7.1 antigen and/or B7.2 antigen are then used to clone variable domain sequences for the manufacture of privatized antibodies essentially as described in Newman et al, (1992), Id. and Newman et al, U.S. Serial No. 379,072, filed January 25, 1995, corresponding to PCT/US92/06194, which was published as WO93/02108. Essentially, this entrails extraction of RNA therefrom, conversion to cDNA, and amplification, thereof by PCR using Ig specific primers. Suitable primers are described in Newman et al, 1992, Id. and in U.S. Serial No. 379,072. (See, in particular, Figure 1 of U.S. Serial No. 379,072).

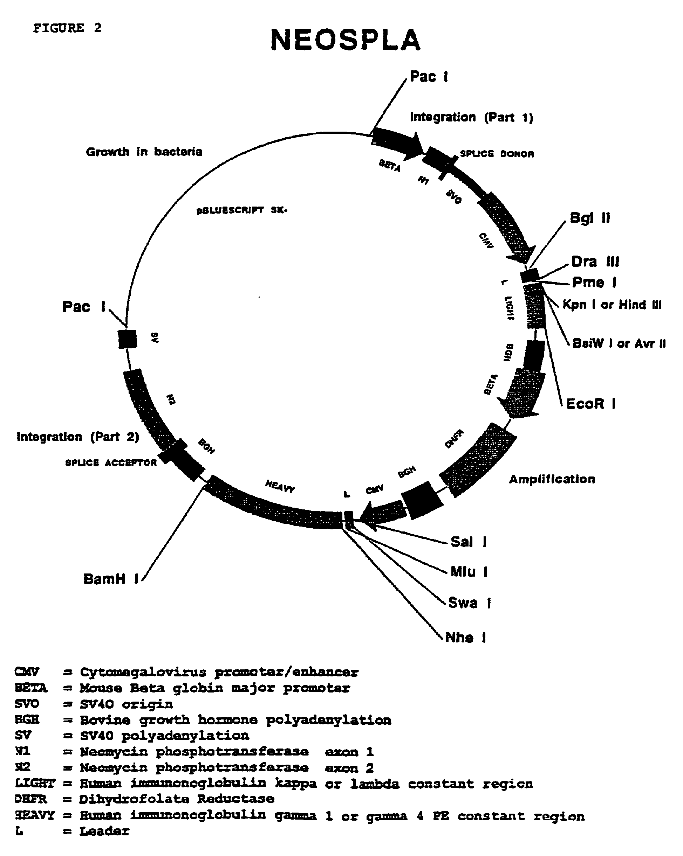

The cloned monkey variable genes are then inserted into an expression vector which contains human heavy and light chain constant region genes. Preferably, this is effected using a proprietary expression vector of IDEC, Inc., referred to as NEOSPLA. This vector is shown in Figure 2 and contains the cytomegalovirus promoter/enhancer, the mouse beta globin major promoter, the SV40 origin of replication, the bovine growth hormone polyadenylation sequence, neomycin phosphotransferase exon 1 and exon 2, human immunoglobulin kappa or lambda constant region, the dihydrofolate reductase gene, the human immunoglobulin gamma 1 or gamma 4 PE constant region and leader sequence. This vector has been found to result in very high level expression of primatized antibodies upon incorporation of monkey variable region genes, transfection in CHO cells, followed by selection in G418 containing medium and methotrexate amplification.

For example, this expression system has been previously disclosed to result in primatized antibodies having high avidity (Kd ≤ 10¹⁰ M) against CD4 and other human cell surface receptors. Moreover, the antibodies have been found to exhibit the same affinity, specificity and functional activity as the original monkey antibody. This vector system is substantially disclosed in commonly assigned U.S. Serial No. 379,072, corresponding to PCT/US92/06194 which was published as WO93/02108 as well as U.S. Serial No. 08/149,099, filed on November 3, 1993, corresponding to PCT/US93/10953 which was published as WO94/11026. This system provides for high expression levels, i.e., > 30 pg/cell/day.

As discussed *infra,* the subject inventors have selected four lead candidate monkey monoclonal antibodies which specifically bind the B7.1 antigen. These monkey monoclonal antibodies are referred to herein as 7B6, 16C10, 7C10 and 20C9.

As discussed in greater detail infra, these antibodies were evaluated for their ability to block B cell/T cell interactions as measured by IL-2 production and tritiated thymidine uptake in a mixed lymphocyte reaction for T cell binding experiments for T cell binding, human buffy coat peripheral blood lymphocytes were cultured for 3-6 days in the presence of PHA stimulator. B7 binding was radioassayed using ¹²⁵I-radiolabeled soluble B7.1. The observed results indicate that all of these antibodies bind B7.1 antigen with high affinity and effectively block B cell/T cell interactions as evidenced by reduced IL-2 production and reduced proliferation of mixed lymphocyte cultures.

The properties of these particular monkey monoclonal antibodies are summarized below:
1. Scatchard analysis showed that the apparent affinity constants (Kd) for the monkey antibodies binding to B7-Ig coated plates were approximated to be:

| | | |
|---|---|---|
| a: | 7C10: | 6.2 x 10⁻⁹M |
| b: | 16C10: | 8.1 x 10⁻⁹M |
| c: | 7B6 : | 10.7 x 10⁻⁹M |
| d: | 20C9: | 16.8 x 10⁻⁹M |

2. The antibodies were tested in vitro in a mixed lymphocyte reaction assay (MLR). The MLR showed that all 4 anti-B7.1 antibodies inhibit IL-2 production to different extents as shown by the following Ic₅₀ values:

| | | |
|---|---|---|
| a: | 7B6: | 5.0 µg/M |
| b: | 16C10: | <0.1 µg/M |
| c: | 20C9: | 2.0 µg/M |
| d: | 7C10: | 5.0 µg/M |

3. The monkey anti-B7.1 antibodies were tested for their ability to bind B7 on human peripheral blood lymphocytes (PBL). FACS analysis showed that all 4 monkey antibodies tested positive.
4. Monkey antibodies 16C10, 7B6, 7C10 and 20C9 were tested for C1q binding by FACS analysis. Results showed 7C10 monkey Ig had strong human C1q binding after incubating with B7.1 CHO-transfected cells. 16C10 was positive, while 20C9 and 7B6 monkey antibodies were negative.
5. To select an animal model for patch-tox studies, the monkey antibodies were tested with animal blood from different species. It was determined that the monkey anti-B7.1 antibodies cross-reacted with human, chimpanzee.

Based on these properties, it would appear that three monkey monoclonal antibodies possess the most advantageous properties, 16C10, 7C10 and 20C9, with 16C10 and 7C10 being somewhat better than 20C9.

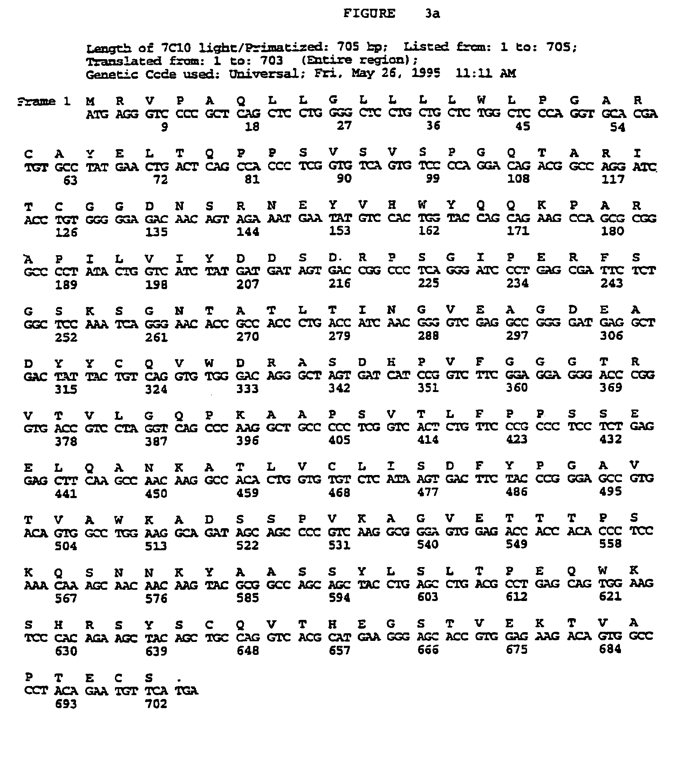

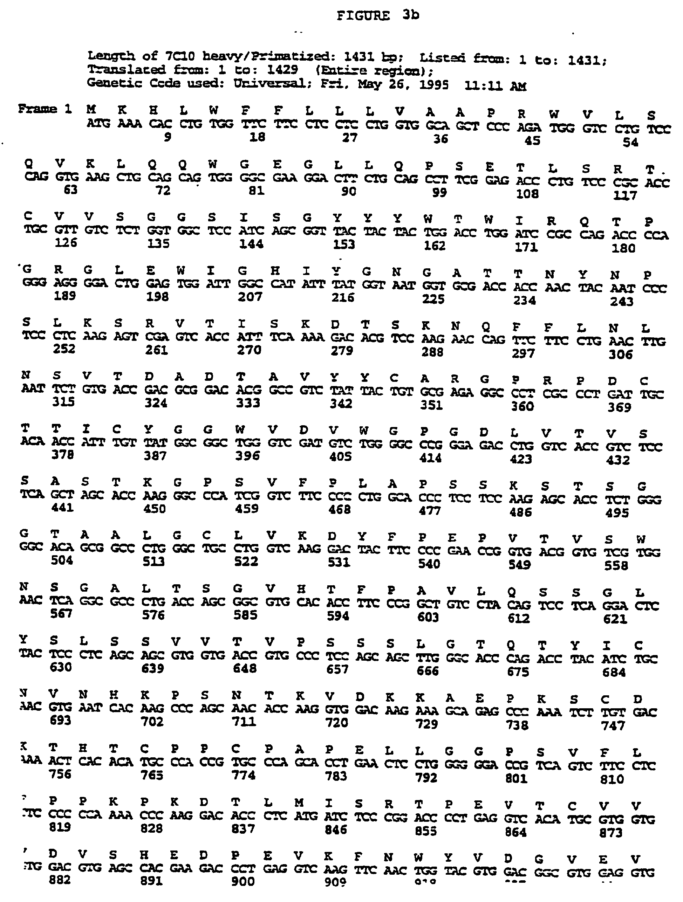

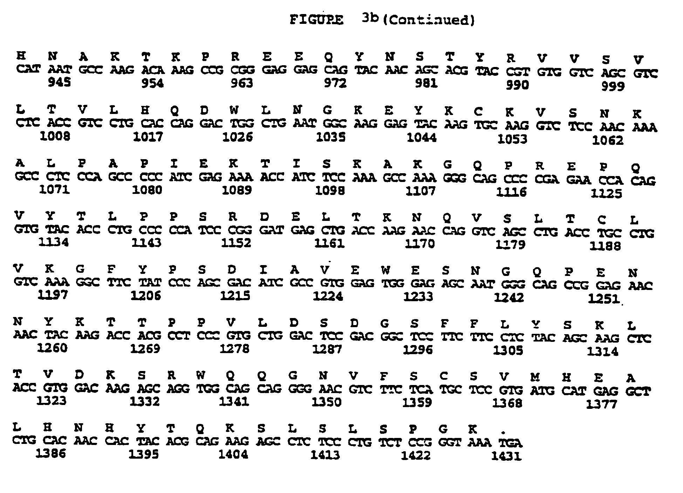

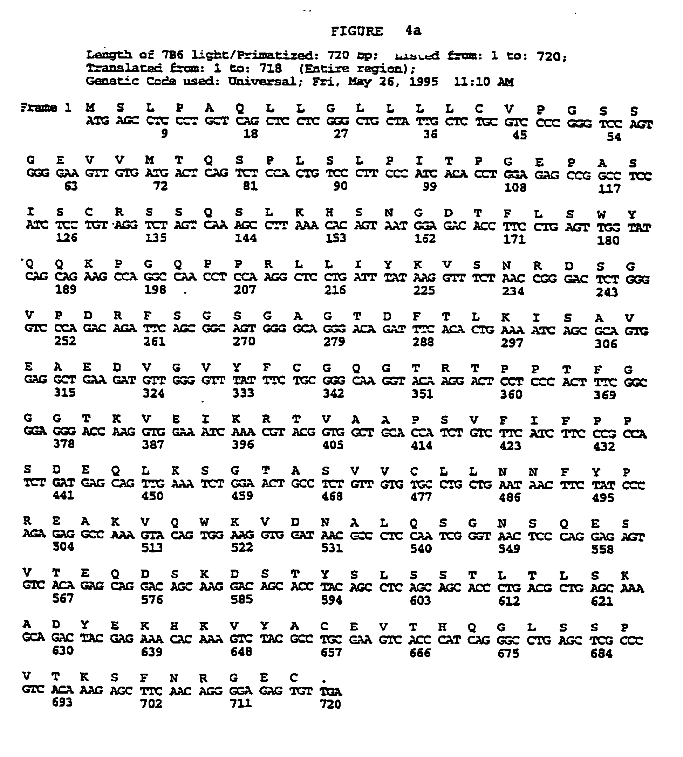

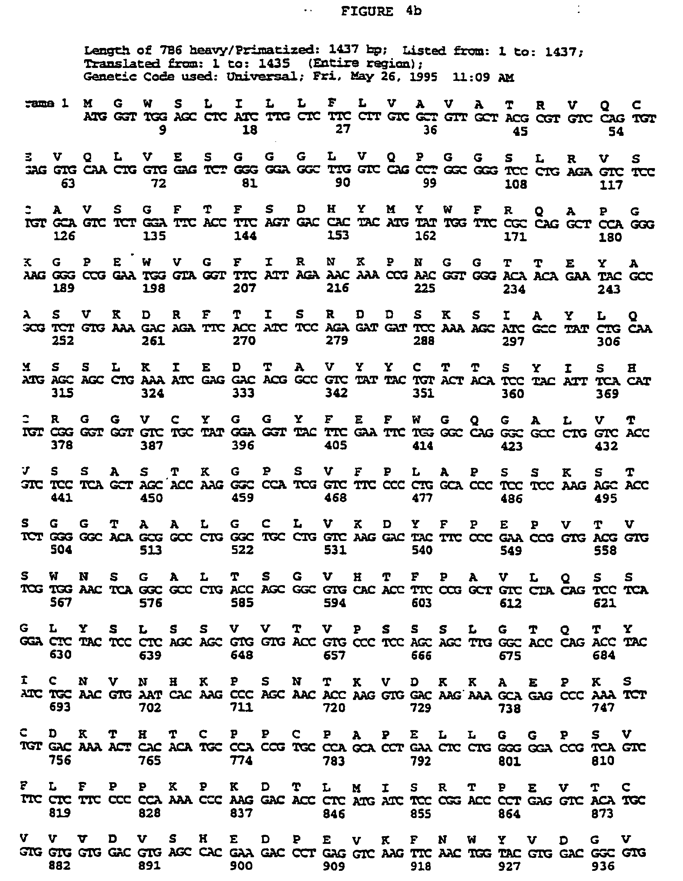

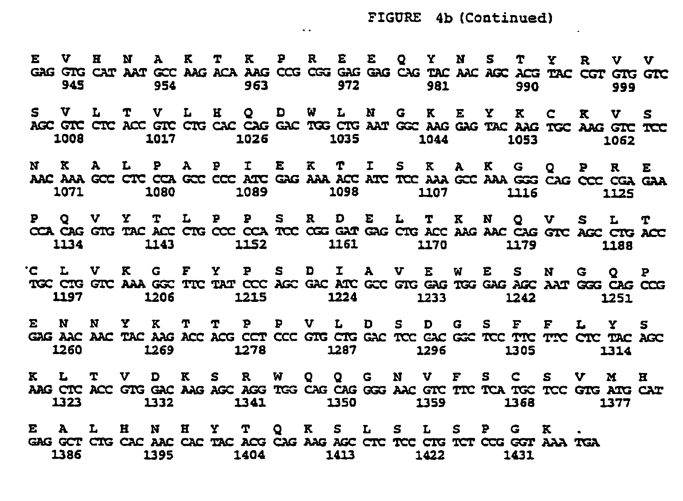

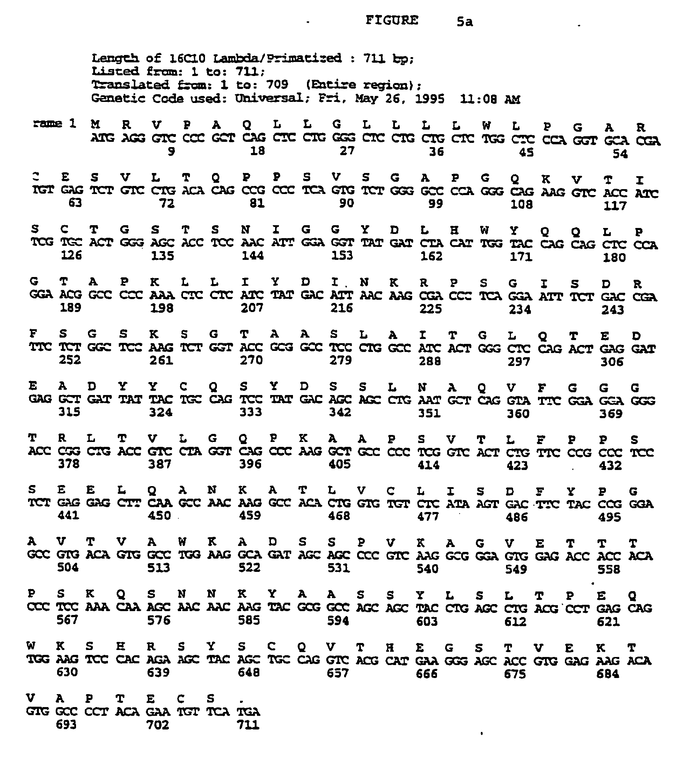

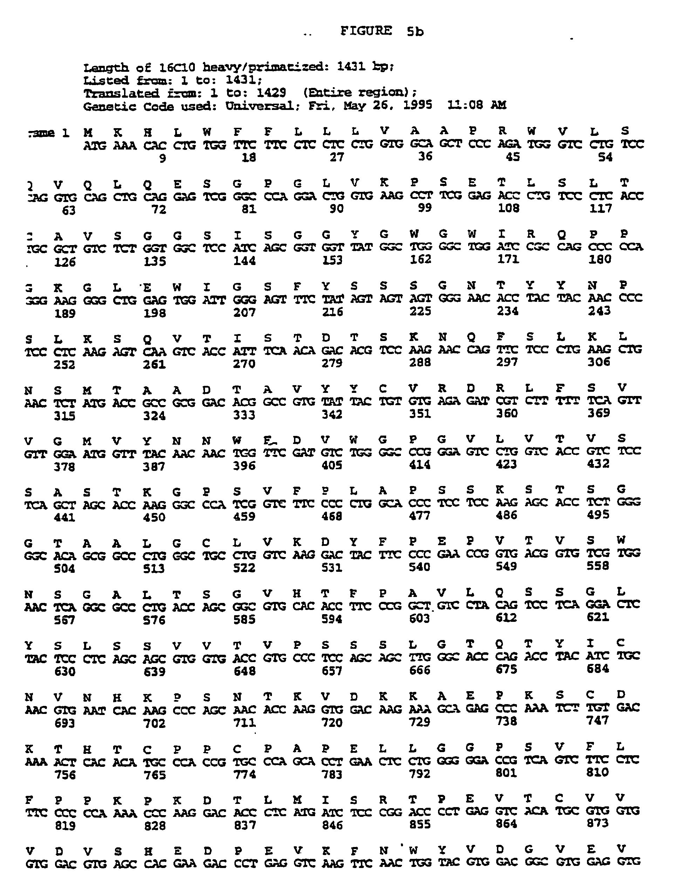

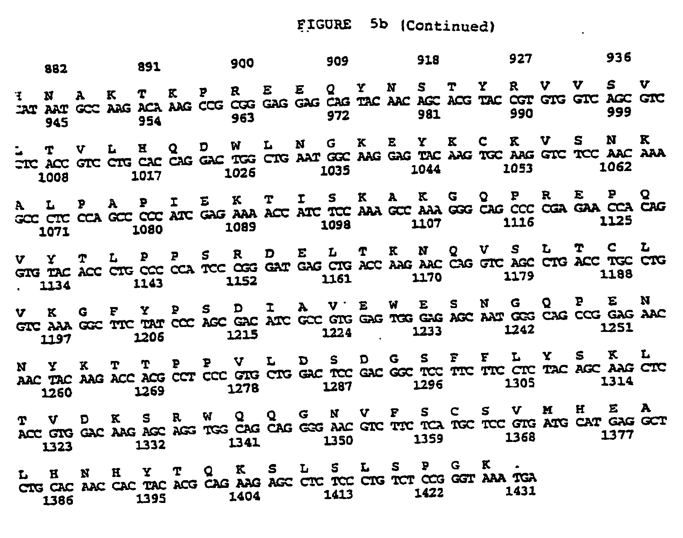

Using the techniques described supra, and in commonly assigned U.S. Serial No. 08/379,072, the present inventors have cloned the variable domains of 7C10, 7B6 and 16C10, and provide the amino acid and nucleic acid sequences of primatized forms of the 7C10 light chain, 7C10 heavy chain, 7B6 light chain, 7B6 heavy chain, 16C10 light chain and 16C10 heavy chain. These amino acid and nucleic acid sequences may be found in Figures 3a and 3b, 4a and 4b, and 5a and 5b. The DNA and amino acid sequence for the human gamma 1, gamma 4 constant domain may be found in 08/379, 072.

As discussed supra, these primatized antibodies are preferably expressed using the NEOSPLA expression vector shown in Figure 2 which is substantially described in commonly assigned 08/379,072 and 08/149,099, both of which applications are incorporated by reference herein.. commonly assigned 08/379,072 (corresponding to PCT/US92/06194, which was published as WO93/02108) and 08/149,099 (corresponding to PCT/US93/10953, which was published as WO94/11026).

As previously noted, the subject primatized antibodies will preferably contain either the human immunoglobulin gamma 1 or gamma 4 constant region, with gamma 4 preferably mutated at two positions to create gamma 4 PE. The gamma 4 PE mutant contains two mutations, a glutamic acid in the CH2 region introduced to eliminate residual FCR binding, and a proline substitution in the hinge region, intended to enhance the stability of the heavy chain disulfide bond interaction. (*See*, Alegre et al, J. Immunol., 148, 346i-3468, (1992); and Angel, et al, Mol. Immunol., 30, 105-158, (1993),

Whether the subject primatized antibodies contain the gamma 1, gamma 4 or gamma 4 PE constant region largely depends on the particular disease target. Preferably, depleting and non-depleting privatized. IgG1 and IgG4 antibodies are created and tested against specific disease targets.

Given the described binding and functional properties of the subject monkey monoclonal antibodies, these anti-B7.1 monoclonal antibodies and primatized forms thereof should be well suited as therapeutic agents for blocking the B7:CD28 interaction thereby providing for immunosuppression. In particular, given their high affinity to B7.1 antigen and ability to block B cell/T cell interactions as measured by IL-2 production and tritiated thymidine uptake in mixed lymphocyte culture as well as their ability to effectively inhibit antigen driven responses in donor spleen cell cultures as shown by reduced antigen specific IgG responses, IL-2 production and cell proliferation, these monkey monoclonal antibodies and primatized forms thereof should function as effective immunosuppressants which modulate the B7:CD28 pathway. This is significant for the treatment of many diseases wherein immunosuppression is therapeutically desirable, e.g., autoimmune diseases, to inhibit undesirable antigen specific IgG responses, and also for prevention of organ rejection and graft-versus-host disease. Essentially, the subject antibodies will be useful in treating any disease wherein suppression of the B7:CD28 pathway is therapeutically desirable.

Key therapeutic indications for the subject anti-B7.1 antibodies include, by way of example, autoimmune diseases such as idiopathic thrombocytopenia purpura (ITP), systemic lupus erythematosus (SLE), type 1 diabetes mellitus, multiple sclerosis, aplastic anemia, psoriasis, allergy, inflammatory bile disease and rheumatoid arthritis.

Another significant therapeutic indication of the subject anti-B7.1 antibodies is for prevention of graft-versus-host-disease (GVHD) during organ transplant and bone marrow transplant (BMT). The subject antibodies may be used to induce host tolerance to donor-specific alloantigens and thereby facilitate engraftment and reduce the incidence of graft rejection. It has been shown in a murine model of allogeneic cardiac transplantation that intravenous administration of CTLA4-Ig can result in immunosuppression or even induction of tolerance to alloantigen. (Lin et al, J. Exp. Med. 178:1801, 1993; Torka et al, Proc. Natl. Acad. Sci.. USA, 89:11102, 1992). It is expected that the subject primatized anti-B7.1 antibodies will exhibit similar or greater activity.

Antibodies produced in the manner described above, or by equivalent techniques, can be purified by a combination of affinity and size exclusion chromatography for characterization in functional biological assays. These assays include determination of specificity and binding affinity as well as effector function associated with the expressed isotype, e.g., ADCC, or complement fixation. Such antibodies may be used as passive or active therapeutic agents against a number of human diseases, including B cell lymphoma, infectious diseases including viral diseases such as HIV/AIDS, autoimmune and inflammatory diseases, and transplantation. The antibodies can be used either in their native form, or as part of an antibody/chelate, antibody/drug or antibody/toxin complex. Additionally, whole antibodies or antibody fragments (Fab₂, Fab, Fv) may be used as imaging reagents or as potential vaccines or immunogens in active immunotherapy for the generation of anti-idiotypic responses.

The amount of antibody useful to produce a therapeutic effect can be determined by standard techniques well known.to those of ordinary skill in the art. The antibodies will generally be provided by standard technique within a pharmaceutically acceptable buffer, and may be administered by any desired route. Because of the efficacy of the presently claimed antibodies and their tolerance by humans it is possible to administer these antibodies repetitively in order to combat various diseases or disease states within a human.

The anti-B7.1 antibodies (or fragments thereof) of this invention are useful for inducing immunosuppression, i.e., inducing a suppression of a human's or animal's immune system. This invention therefore relates to the manufacture of a medicament for use in a method of prophylactically or therapeutically inducing immunosuppression in a human or other animal in need thereof by administering an effective, non-toxic amount of such an antibody of this invention to such human or other animal.

The ability of the compounds of this invention to induce immunosuppression has been demonstrated in standard tests used for this purpose, for example, a mixed lymphocyte reaction test or a test measuring inhibition of T-cell proliferation measured by thymidine uptake.

The fact that the antibodies of this invention have utility in inducing immunosuppression indicates that they should be useful in the treatment or prevention of resistance to or rejection of transplanted organs or tissues (e.g., kidney, heart, lung, bone marrow, skin, cornea, etc.); the treatment or prevention of autoimmune, inflammatory, proliferative and hyperproliferative diseases, and of cutaneous manifestations of immunologically medicated diseases (e.g., rheumatoid arthritis, lupus erythematosus, systemic lupus erythematosus, Hashimotos thyroiditis, multiple sclerosis, myasthenia gravis, type 1 diabetes, uveitis, nephrotic syndrome, psoriasis, atopical dermatitis, contact dermatitis and further eczematous dermatitides, seborrheic dermatitis, Lichen planus, pemphigus, bullous pemphigus, Epidermolysis bullosa, urticaria, angioedemas, vasculitides, erythema, cutaneous eosinophilias, Alopecia areata, etc.); the treatment of reversible obstructive airways disease, intestinal inflammations and allergies (e.g., inflammatory bile disease, Coeliac disease, proctitis, eosinophilia gastroenteritis, mastocytosis, Crohn's disease and ulcerative colitis), food-related allergies (e.g., migraine, rhinitis and eczema), and other types of allergies.

One skilled in the art would be able, by routine experimentation, to determine what an effective, non-toxic amount of antibody would be for the purpose of inducing immunosuppression. Generally, however, an effective dosage will be in the range of about 0.05 to 100 milligram per kilogram body weight per day.

The antibodies (or fragments thereof) of this invention should also be useful for treating tumors in a mammal. More specifically, they should be useful for reducing tumor size, inhibiting tumor growth and/or prolonging the survival time of tumor-bearing animals. Accordingly, this invention also relates to the manufacture of a medicament for use in a method of treating tumors in a human or other animal by administering to such human or animal an effective, non-toxic amount of an antibody. One skilled in the art would be able, by routine experimentation, to determine what an effective, non-toxic amount of anti-B7 antibody would be for the purpose of treating carcinogenic tumors. Generally, however, an effective dosage is expected to be in the range of about 0.05 to 100 milligrams per kilogram body weight per day.

The antibodies of the invention may be administered to a human or other animal in accordance with the aforementioned methods of treatment in an amount sufficient to produce such effect to a therapeutic or prophylactic degree. Such antibodies of the invention can be administered to such human or other animal in a conventional dosage form prepared by combining the antibody of the invention with a conventional pharmaceutically acceptable carrier or diluent according to known techniques. It will be recognized by one of skill in the art that the form and character of the pharmaceutically acceptable carrier or diluent is dictated by the amount of active ingredient with which it is to be combined, the route of administration and other well-known variables.

The route of administration of the antibody (or fragment thereof) of the invention may be oral, parenteral, by inhalation or topical. The term parenteral as used herein includes intravenous, intraperitoneal, intramuscular, subcutaneous, rectal or vaginal administration. The subcutaneous and intramuscular forms of parenteral administration are generally preferred.

The daily parenteral and oral dosage regimens for employing compounds of the invention to prophylactically or therapeutically induce immunosuppression, or to therapeutically treat carcinogenic tumors will generally be in the range of about 0.05 to 100, but preferably about 0.5 to 10, milligrams per kilogram body weight per day.

The antibodies of the invention may also be administered by inhalation. By "inhalation" is meant intranasal and oral inhalation administration. Appropriate dosage forms for such administration, such as an aerosol formulation or a metered dose inhaler, may be prepared by conventional techniques. The preferred dosage amount of a compound of the invention to be employed is generally within the range of about 10 to 100 milligrams.

The antibodies of the invention may also be administered topically. By topical administration is meant non-systemic administration and includes the application of an antibody (or fragment thereof) compound of the invention externally to the epidermis, to the buccal cavity and instillation of such an antibody into the ear, eye and nose, and where it does not significantly enter the blood stream. By systemic administration is meant oral, intravenous, intraperitoneal and intramuscular administration. The amount of an antibody required for therapeutic or prophylactic effect will, of course, vary with the antibody chosen, the nature and severity of the condition being treated and the animal undergoing treatment, and is ultimately at the discretion of the physician. A suitable topical dose of an antibody of the invention will generally be within the range of about 1 to 100 milligrams per kilogram body weight daily.

### Formulations

While it is possible for an antibody or fragment thereof to be administered alone, it is preferable to present it as a pharmaceutical formulation. The active ingredient may comprise, for topical administration, from 0.001% to 10% w/w, e.g., from 1% to 2% by weight of the formulation, although it may comprise as much as 10% w/w but preferably not in excess of 5% w/w and more preferably from 0.1% to 1% w/w of the formulation.

The topical formulations of the present invention, comprise an active ingredient together with one or more acceptable carrier(s) therefor and optionally any other therapeutic ingredients(s). The carrier(s) must be "acceptable" in the sense of being compatible with the other ingredients of the formulation and not deleterious to the recipient thereof.

Formulations suitable for topical administration include liquid or semi-liquid preparations suitable for penetration through the skin to the site of where treatment is required, such as liniments, lotions, creams, ointments or pastes, and drops suitable for administration to the eye, ear or nose.

Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent. The resulting solution may then be clarified by filtration, transferred to a suitable container which is then sealed and sterilized by autoclaving or maintaining at 90°-100°C for half an hour. Alternatively, the solution may be sterilized by filtration and transferred to the container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Lotions according to the present invention include those suitable for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy basis. The basis may comprise hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as stearic or oleic acid together with an alcohol such as propylene glycol or macrogols. The formulation may incorporate any suitable surface active agent such as an anionic, cationic or non-ionic surface active such as sorbitan esters or polyoxyethylene derivatives thereof. Suspending agents such as natural gums, cellulose derivatives or inorganic materials such as silicaceous silicas, and other ingredients such as lanolin, may also be included.

The subject anti-B7.1 antibodies or fragments thereof may also be administered in combination with other moieties which modulate the B7:CD28 pathway. Such moieties include, by way of example, cytokines such as IL-7 and IL-10, CTLA4-Ig, soluble CTLA-4 and anti-CD28 antibodies and fragments thereof. Also, the subject antibodies may be administered in combination with other immunosuppressants. Such immunosuppressants include small molecules such as cyclosporin A (CSA) and FK506; monoclonal antibodies such as anti-tumor necrosis factor a (anti-TNFa), anti-CD54, anti-CD11, anti-CD11a, and anti-IL-1; and, other soluble receptors such as rTNFa and rIL-1.

It will be recognized by one of skill in the art that the optimal quantity and spacing of individual dosages of an antibody or fragment thereof of the invention will be determined by the nature and extent of the condition being treated, the form, route and site of administration, and the particular animal being treated, and that such optimums can be determined by conventional techniques. It will also be appreciated by one of skill in the art that the optimal course of treatment, i.e., the number of doses of an antibody or fragment thereof of the invention given per day for a defined number of days, can be ascertained by those skilled in the art using conventional course of treatment determination tests.

Without further elaboration, it is believed that one skilled in the art can, using the preceding description, utilize the present invention to its fullest extent. The following formulations are, therefore, to be construed as merely illustrative embodiments and not a limitation of the scope of the present invention in any way.

### Capsule Composition

A pharmaceutical composition of this invention in the form of a capsule is prepared by filling a standard two-piece hard gelatin capsule with 50 mg. of an antibody or fragment thereof of the invention, in powdered form, 100 mg. of lactose, 32 mg. of talc and 8 mg. of magnesium stearate.

### Injectable Parenteral Composition

A pharmaceutical composition of this invention in a form suitable for administration by injection is prepared by stirring 1.5% by weight of an antibody or fragment thereof of the invention in 10% by volume propylene glycol and water. The solution is sterilized by filtration.

### Ointment Composition

Antibody or fragment thereof of the invention 1.0 g.

White soft paraffin to 100.0 g.

The antibody or fragment thereof of the invention is dispersed in a small volume of the vehicle to produce a smooth, homogeneous product. Collapsible metal tubes are then filled with the dispersion.

### Topical Cream Composition

Antibody or fragment thereof of the invention 1.0 g.

Polawax GP 200 20.0 g.

Lanolin Anhydrous 2.0 g.

White Beeswax 2.5 g.

Methyl hydroxybenzoate 0.1 g.

Distilled Water to 100.0 g.

The polawax, beeswax and lanolin are heated together at 60°C. A solution of methyl hydroxybenzoate is added and homogenization is achieved using high speed stirring. The temperature is then allowed to fall to 50°C. The antibody or fragment thereof of the invention is then added and dispersed throughout, and the composition is allowed to cool with slow speed stirring.

### Topical Lotion Composition

Antibody or fragment thereof of the invention 1.0 g.

Sorbitan Monolaurate 0.6 g.

Polysorbate 20 0.6 g.

Cetostearyl Alcohol 1.2 g.

Glycerin 6.0 g.

Methyl Hydroxybenzoate 0.2 g.

Purified Water B.P. to 100-00 ml. (B.P. = British Pharmacopeia)

The methyl hydroxybenzoate and glycerin are dissolved in 70 ml. of the water at 75°C.e.The sorbitan monolaurate, polysorbate 20 and cetostearyl alcohol are melted together at 75°C and added to the aqueous solution. The resulting emulsion is homogenized, allowed to cool with continuous stirring and the antibody or fragment thereof of the invention is added as a suspension in the remaining water. The whole suspension is stirred until homogenized.

### Eye Drop Composition

Antibody or fragment thereof of the invention 0.5 g.

Methyl Hydroxybenzoate 0.01 g.

Propyl Hydroxybenzoate 0.04 g.

Purified Water B.P. to 100-00 ml.

The methyl and propyl hydroxybenzoates are dissolved in 70 ml. purified water at 75°C and the resulting solution is allowed to cool. The antibody or fragment thereof of the invention is then added, and the solution is sterilized by filtration through a membrane filter (0.022 µm pore size), and packed aseptically into suitable sterile containers.

### Composition for Administration by Inhalation

For an aerosol container with a capacity of 15-20 ml: mix 10 mg. of an antibody or fragment thereof of the invention with 0.2-0.5% of a lubricating agent, such as polysorbate 85 or oleic acid, and disperse such mixture in a propellant, such as freon, preferably in a combination of (1,2 dichlorotetrafluoroethane) and difluorochloro-methane and put into an appropriate aerosol container adapted for either intranasal or oral inhalation administration.

### Composition for Administration by Inhalation

For an aerosol container with a capacity of 15-20 ml: dissolve 10 mg. of an antibody or fragment thereof of the invention in ethanol (6-8 ml.), add 0.1-0.2% of a lubricating agent, such as polysorbate 85 or oleic acid; and disperse such in a propellant, such as freon, preferably in combination of (1.2 dichlorotetrafluoroethane) and difluorochloromethane, and put into an appropriate aerosol container adapted for either intranasal or oral inhalation administration.

The antibodies and pharmaceutical compositions of the invention are particularly useful for parenteral administration, i.e., subcutaneously, intramuscularly or intravenously. The compositions for parenteral administration will commonly comprise a solution of an antibody or fragment thereof of the invention or a cocktail thereof dissolved in an acceptable carrier, preferably an aqueous carrier. A variety of aqueous carriers may be employed, e.g., water, buffered water, 0.4% saline, 0.3% glycine, and the like. These solutions are sterile and generally free of particulate matter. These solutions may be sterilized by conventional, well-known sterilization techniques. The compositions may contain pharmaceutically acceptable auxiliary substances as required to approximate physiological conditions such as pH adjusting and buffering agents, etc. The concentration of the antibody or fragment thereof of the invention in such pharmaceutical formulation can vary widely, i.e., from less than about 0.5%, usually at or at least about 1% to as much as 15 or 20% by weight, and will be selected primarily based on fluid volumes, viscosities, etc., according to the particular mode of administration selected.

Thus, a pharmaceutical composition of the invention for intramuscular injection could be prepared to contain 1 Ml sterile buffered water, and 50 mg. of an antibody or fragment thereof of the invention. Similarly, a pharmaceutical composition of the invention for intravenous infusion could be made up to contain 250 ml. of sterile Ringer's solution, and 150 mg of an antibody or fragment thereof of the invention. Actual methods for preparing parenterally administrable compositions are well known or will be apparent to those skilled in the art, and are described in more detail in, for example, Remington's Pharmaceutical Science, 15th ed., Mack Publishing Company, Easton, Pennsylvania.

The antibodies (or fragments thereof) of the invention can be lyophilized for storage and reconstituted in a suitable carrier prior to use. This technique has been shown to be effective with conventional immune globulins and art-known lyophilization and reconstitution techniques can be employed.

Depending on the intended result, the pharmaceutical composition of the invention can be administered for prophylactic and/or therapeutic treatments. In therapeutic application, compositions are administered to a patient already suffering from a disease, in an amount sufficient to cure or at least partially arrest the disease and its complications. In prophylactic Applications, compositions containing the present antibodies or a cocktail thereof are administered to a patient not already in a disease state to enhance the patient's resistance.

Single or multiple administrations of the pharmaceutical compositions can be carried out with dose levels and pattern being selected by the treating physician. In any event, the pharmaceutical composition of the invention should provide a quantity of the altered antibodies (or fragments thereof) of the invention sufficient to effectively treat the patient.

It should also be noted that the antibodies of this invention may be used for the design and synthesis of either peptide or non-peptide compounds (mimetics) which would be useful in the same therapy as the antibody. See, e.g., Saragovi et al., Science, 253, 792-795 (1991).

To further illustrate the invention, the following examples are provided. These examples are not intended, nor are they to be construed, as further limiting the invention.

### Example 1

Recombinant immunoglobulin libraries displayed on the surface of filamentous phage were first described by McCafferty et al, Nature, 348:552-554, 1990 and Barbas et al, Proc. Natl. Acad. Sci., USA 88:7978-7982, 1991. Using this technology, high affinity antibodies have been isolated from immune human recombinant libraries (Barbas et al, Proc. Natl. Acad. Sci., USA 589:10164-10168, 1992). Although the phage display concept used is substantially similar to that described by Barbas, 1991, Id. the technique has been modified by the substitution of a unique vector for monkey libraries to reduce the possibility of recombination and improve stability. This vector, pMS, Figure 1 contains a single lac promoter/operator for efficient transcription and translation of polycistronic heavy and light chain monkey DNA. This vector contains two different leader sequences, the omp A (Movva et al, J. Biol. Chem., 255: 27-29, (1980), for the light chain and the pel B (Lei, J. Bact., 4379-109:4383 (1987) for the heavy chain Fd. Both leader sequences are translated into hydrophobic signal peptides that direct the secretion of the heavy and light chain cloned products into the periplasmic space. In the oxidative environment of the periplasm, the two chains fold and disulfide bonds form to create stable Fab fragments. We derived the backbone of the vector from the phagemid bluescript. (Stratagene, La Jolla, CA). It contains the gene for the enzyme beta-lactamase that confers ampicillin (carbenicillin) resistance to bacteria that harbor pMS DNA. We also derived, from bluescript, the origin of replication of the multicopy plasmid ColEl and the origin of replication of the filamentous bacteriophage fl. The origin of replication of phage fl (the so-called intragenic region), signals the initiation of synthesis of single stranded pMS DNA, the initiation of capsid formation and the termination of RNA synthesis by viral enzymes. The replication and assembly of pMS DNA strands into phage particles requires viral proteins that must be provided by a helper phage. We have used helper phage VCSM13 which is particularly suited for this, since it also contains a gene coding for kanamycin resistance. Bacteria infected with VCSM13 and pMS can be selected by adding both kanamycin and carbenicillin to the growth medium. The bacteria will ultimately produce filamentous phage particles containing either pMS or VCSM13 genomes. Packaging of the helper phage is less efficient than that of pMS, resulting in a mixed phage population that contains predominately recombinant pMS phages. The ends of the phage pick up minor coat proteins specific to each end. Of particular interest here is the gene III product which is present in three to five copies at one end of the phage. The gene III product is 406 amino acid residues and is required for phage infection of E. coli via the F pili. The first two domains of the heavy chain, the variable and the CH1 domain, are fused to the carboxy-terminal half of the gene III protein. This recombinant pili protein, directed by the pel B leader, is secreted to the peroplasm where it accumulates and forms disulfide bonds with the light chain before it is incorporated in the coat of the phage. Also, another vector contains a FLAG sequence engineered downstream of the gene III. The FLAG is an 8 amino acid peptide expressed at the carboxy terminal of the Fd protein. We are using commercially available monoclonal anti-FLAG M2 for both purification and detection of phage Fab by ELISA (Brizzard, Bio Techniques, 16 (4) :730-731, (1994)).

After constructing the vector pMS, we tested its ability to produce phage bound Fab using control antibody genes. We cloned an anti-tetanus toxoid antibody, (obtained from Dr. Carlos Barbas), into pMS and transformed XLI-blue. We co-infected our cells with VCSM13 and generated phage displaying the anti-tetanus toxoid antibody. We performed efficiency experiments where anti-tetanus toxoid phage were combined with phage beading an irrelevant antibody at 1:100,000. We performed three rounds of panning by applying 50 µl of the mixed phage to antigen (tetanus toxoid) coated polystyrene wells. Non-adherent phage were washed off and the adherent phage were eluted with acid. The eluted phage were used to infect a fresh aliquot of XL1-Blue bacteria and helper phage was added. After overnight amplification, phage were prepared and again panned on antigen coated plates. After three rounds of panning, we were able to show that we had successfully enriched for the anti-tetanus toxoid phage. The success of this technology also depends on the ability to prepare soluble Fabs for characterization of the final panned product. This was achieved by excising gene III from the pMS DNA using the restriction enzyme Nhe I followed by re-ligation. After the gene III was excised, the Fab was no longer displayed on the phage surface but accumulated in the piroplasmic space. Lysates were prepared from bacteria expressing soluble Fab and tested for antigen specificity using an ELISA. High levels of soluble Fab were detected.

In order to adapt phage display technology for use with macaque libraries, we developed specific primers for PCR amplifying monkey immunoglobulin genes. These were based on macaque sequences we obtained while developing the PAIMATIZED^{®} antibody technology (See, 08/379,072 (corresponding to PCT/US92/06194 which was published as WO93/02108) and databases containing human sequences. (Kabat et al, (1991), "Sequences of Proteins of Immunological Interest," U.S. Dept. of Health and Human Services, National Institute of Health).

We developed three sets of primers to cover amplification of the macaque repertoire. Our first set of primers was designed for amplification of the heavy chain VH and CH1 (Fd) domains. It, consisted of a 3' -CH1 domain primer and six 5' VH family specific primers that bind in the framework 1 region. Our second set of primers, for amplifying the whole lambda chain, covers the many lambda chain subgroup. It consists of a 3' primer and three 5' degenerate primers that bind in the VL framework 1 region. Our third set of primers was designed for amplification of the kappa chain subgroups. It consists of one 3' primer and five VK framework 1 primers. Using each of these sets, PCR parameters were optimized to obtain strong enough signals from each primer pair so that ample material was available for cloning of the library. We recently created macaque combinatorial libraries in our pMS vector using these optimized PCR conditions. Bone marrow biopsies were taken from CD4 immune monkeys as the source of immunoglobulin RNA. The libraries contained approximately 10⁶ members and are currently being panned or specific binders on antigen coated wells.

### Example 2

### Development of B7/CTLA-4 Reagents

We have generated a number of reagents for the purpose of immunizing monkeys, developing binding and functional assays *in vitro*, screening heterohybridomas and panning phage libraries. Table 1 lists each reagent and its intended purpose. In the case of B7.1, RNA was extracted from SB cells and converted to cDNA using reverse transcriptase. The first strand cDNA was PCR amplified using B7.1 specific primers and cloned into IDEC's NEOSPLA mammalian expression vectors. CHO cells were transfected with B7.1 NEOSPLA DNA and clones expressing membrane associated B7.1 were identified. The B7.1 fusion protein was generated similarly, except that the PCR amplified B7.1 gene was cloned into a NEOSPLA cassette vector containing the human CH2 and CH3 immunoglobulin genes. CHO cells were transformed with the B7.1/Ig NEOSPLA DNA and stable clones secreting B7.1/Ig fusion protein were amplified. In general, the B7.2 and CTLA4 reagents were generated in the same manner, except that for B7.2 the RNA was isolated from human spleen cells that had been stimulated 24 hours with anti-Ig and IL-4, and for the CTLA4 constructs the gene source was PHA activated human T cells.

**Table 1**

| **Reagent** | **Purpose** | **CHO Expression** |
|---|---|---|
| Soluble B7.1 | Immunization, immunoassays | Yes |
| B7.1 Transfectant | Screening, ELISA | Yes |
| B7.1/Ig Fusion Protein | Inhibition studies, panning | Yes |
| B7.2 Transfectant | Screening, ELISA | Yes |
| B7.2/Ig Fusion Protein | Inhibition studies, panning | To be completed |
| CTLA4 Transfectant | Inhibition studies | To be completed |
| CTLA4/Ig | Inhibition studies | To be completed |

The availability of these reagents, together with monoclonal antibodies to B7.1 (L3074) (Becton Dickinson, 1994) and B7.2 (Fun-1 (Engel et al, Blood, 84, 1402-1407, (1994) and purified goat and rabbit antisera, specifically developed to detect monkey Fab fragments, facilitates identification of antibodies having the desired properties.

### Example 3

### Generation of a Phage Display Library

Recombinant phage display libraries are generated from B7.1 and B7.2 immune monkeys. Lymph node and bone marrow biopsies are performed 7-12 days after immunization to harvest RNA rich B cells and plasma cells. RNA is isolated from the lymphocytes using the method described by Chomczynski Anal. Biochem., 162(1), 156-159, (1987). RNA is converted to cDNA using an oligo dT primer and reverse transcriptase. The first strand cDNA is divided into aliquots and PCR amplified using the sets of kappa, lambda, and heavy chain Fd region primers described earlier and either Pfu polymerase (Stratagene, San Diego) or Taq polymerase (Promega, Madison). The heavy chain PCR amplified products are pooled, cut with Xho VSpe I restriction enzymes and cloned into the vector pMS. Subsequently, the light chain PCR products are pooled, cut with Sac I/Xba I restriction enzymes, and cloned to create the recombinant library. XLI-Blue E. coli is transformed with the library DNA and super-infected with VCSM13 to produce the phage displaying antibodies. The library is panned four rounds on polystyrene wells coated with B7.1 or B7.2 antigen. Individual phage clones from each round of panning are analyzed. The pMS vector DNA is isolated and the gene III excised. Soluble Fab fragments are generated and tested in ELISA for binding to B7.1 and B7.2.

### Example 4

### Characterization of Phage Fab Fragments

The monkey phage Fab fragments are characterized for their specificity and the ability to block B7.1-Ig and B7.2-Ig binding to CTLA-4-Ig or CTLA-4 transfected cells. Phage fragments are also characterized for cross-reactivity after first panning for 4 rounds on the B7 species used for immunization in order to select for high affinity fragments. Fab fragments identified from four rounds of panning either on B7.1 or B7.2 antigen coated surfaces are scaled up by infection and grown in 24 hour fermentation cultures of E coli. Fragments are purified by Kodak FLAG binding to a anti-FLAG affinity column. Purified phage Fabs are tested for affinity by an ELISA based direct binding modified Scatchard analysis (Katoh et al, J. Chem. BioEng., 76:451-454, (1993)) using Goat anti-monkey Fab antibodies or anti-FLAG MAb conjugated with horseradish peroxidase. The anti-monkey Fab reagents will be absorbed against human heavy chain constant region Ig to remove any cross-reactivity to B7-Ig. Kd values are calculated for each fragment after measurements of direct binding to B7.1-Ig or B7.2-Ig coated plates.

### Example 5

### Phage Fab Fragment Blocking of CTLA-4/B7 Binding

Fab fragments most effectively blocking the binding of B7-Ig at the lowest concentrations are selected as lead candidates. Selections are made by competing off ¹²⁵I-B7-Ig binding to CTLA-4-Ig or CTLA-4 transfected cells. Additional selection criteria include, blocking of mixed lymphocyte reaction (MLR), as measured by inhibiting 3H-thymidine uptake in responder cells (Azuma et al, J. Exp. Med., 177:845-850,; Azuma et al, Nature, 301:76-79, (1993)) and direct analysis of IL-2 production using IL-2 assay kits. The three or four candidates which are most effective in inhibiting of MLR and CTLA-4 binding assays are chosen for cloning into the above-described mammalian expression vector for transfection into CHO cells and expression of chimeric monkey/human antibodies.

### Example 6

### Generation of Monkey Heterohybridomas

Monkey heterohybridomas secreting monoclonal antibodies are generated from existing immunized animals whose sera tested positive for B7.1 and/or B7.2. Lymph node biopsies are taken from animals positive to either, or both, antigens. The method of hybridoma production is similar to the established method used for the generation of monkey anti-CD4 antibodies (Newman, 1992(Id.)). Monkeys with high serum titers will have sections of inguinal lymph nodes removed under anesthesia. Lymphocytes are washed from the tissue and fused with KH6/B5 heteromyeloma cells (Carrol et al, J. Immunol. Meth., 89:61-72, (1986)) using polyethylene glycol (PEG). Hybridomas are selected on H.A.T. media and stabilized by repeated subcloning in 96 well plates.

Monkey monoclonal antibodies specific for B7.1 antigen are screened for cross-reactivity to B7.2. Monkey anti-B7 antibodies will be characterized for blocking of B7/CTLA-4 binding using the ¹²⁵I-B7-Ig binding assay. Inhibition of MLR by 3H-Thymidine uptake and direct measurement of IL-2 production is used to select three candidates. Two candidates will be brought forward in Phase II studies and expressed in CHO cells while repeating all functional studies. For the purposes of developing an animal model for *in vivo* pharmacology, anti-B7 antibodies will be tested on cells of several animal species. The establishment of an animal model will allow preclinical studies to be carried out for the selected clinical indication.

### Example 7

As discussed supra, using the above heterohybridoma methods, 4 lead monkey anti-B7.1 antibodies have been identified: 16C10, 7B6, 7C10 and 20C9. These antibodies were characterized as follows:
Scatchard analysis showed that the apparent affinity constants (Kd) for the monkey antibodies binding to B7-Ig coated plates were approximated to be:

| | | |
|---|---|---|
| a: | 7C10: | 6.2 x 10⁻⁹M |
| b: | 16C10: | 8.1 x 10⁻⁹M |
| c: | 7B6: | 10.7 x 10⁻⁹M |
| d: | 20C9: | 16.8 x 10⁻⁹M |

The antibodies were tested in vitro in a mixed lymphocyte reaction assay (MLR). The MLR showed that all 4 anti-B7.1 antibodies inhibit IL-2 production to different extents:

| | | |
|---|---|---|
| a: | 7B6: | 5.0 µg/Ml |
| b: | 16C10: | 0.1 µg/Ml |
| c: | 20C9: | 2.0 µg/Ml |
| d: | 7C10: | 5.0 µg/Ml |

The monkey anti-B7.1 antibodies were tested for their ability to bind B7 on human peripheral blood lymphocytes (PBL). FACS analysis showed that all 4 monkey antibodies tested positive.
Monkey antibodies 16C10, 7B6, 7C10 and 20C9 were tested for Clq binding by FACS analysis. Results showed 7C10 monkey Ig had strong human C1q binding after incubating with B7.1 CHO-transfected cells. 16C10 was also positive, while 20C9 and 7B6 monkey antibodies were negative.

### Example 8

Using the primatized antibody methodology incorporated by reference to commonly assigned U.S. Serial No. 08/379,072 corresponding to PCT/US92/06194, which was published as WO93/02108 and using the NEOSPLA vector system shown in Figure 2, the heavy and light variable domains of 7C10, 7B6 and 16C10 were cloned and primatized forms thereof have been synthesized in CHO cells using the NEOSPLA vector system. The amino acid and nucleic acid sequences for the primatized 7C10 light and heavy chain, 7B6 light and heavy chain, and 16C10 light and heavy chain are respectively shown in Figures 3a, 3b, 4a, 4b, 5a and 5b.

### Example 9

### Confirming experiments on the non- cross -reactivity of the CTLA-4 and PRIMATIZED^{®} antibody binding sites on B7.1.

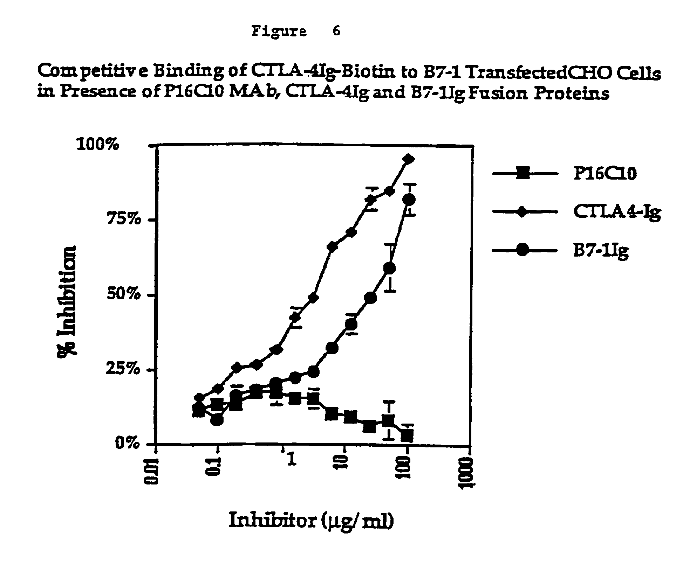

In competitive binding assays using biotinylated CTLA-4Ig (Figure 6), unlabeled primatized 16C10 (i.e., P16C10) was unable to block CTLA-4Ig binding to B7.1 transfected CHO cells. It can be seen that unlabeled CTLA-4Ig and unlabeled B7.1 effectively compete under these conditions.

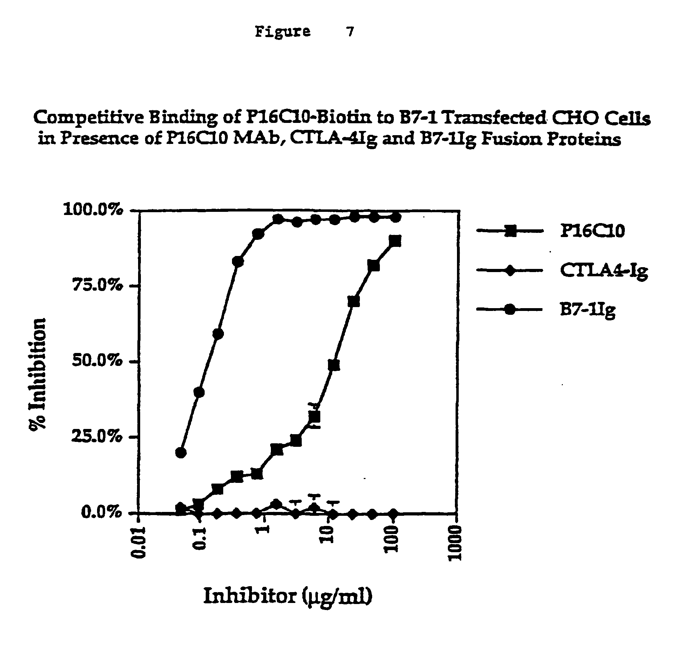

In a second experiment using Biotinylated P16C10, the same conclusions can be made. In the experiment shown in Figure 7, binding of P16C10-Biotin is inhibited by both unlabeled P16C10 and B7.1Ig, but not by CTLA-4Ig. Although CTLA-4Ig is reported to be as much as 4-10 fold higher in affinity (Kd=0.4 nM; Morton et al., J. Immunol. 156:1047-1054 (1996)), there is no significant inhibition of P16C10 binding even at CTLA-4Ig concentrations as high as 100 fold excess.

Similar results were obtained using the primatized antibody 7C10 (P7C10) when it was substituted for P16C10 in the experiments (data not provided).

### Example 10

### Comparing the ability of L307.4 and BB-1 mouse antibodies to bind to B7 CHO cells in the presence of CTLA-4Ig.

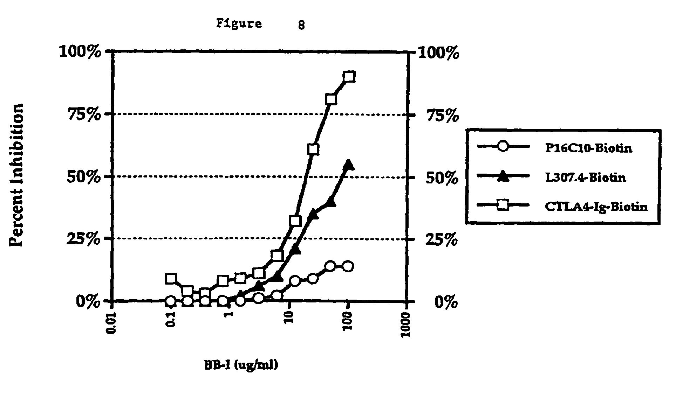

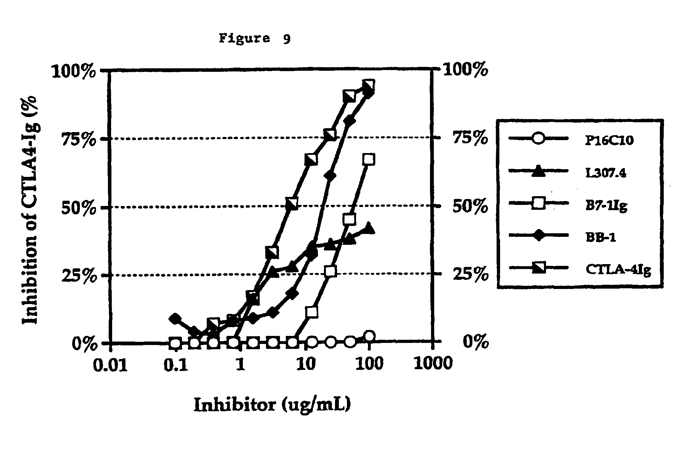

The binding of L307.4 and BB-1 murine anti-B7 antibody in the presence of CTLA-4Ig was studied in order to determine whether the mouse antibody binding sites overlapped with the CTLA-4 binding site. Competition assay experiments using P16C10-Biotin, L307.4-Biotin and CTLA-4Ig-Biotin were done to insure that affinity differences did not prevent detection of competitive binding. The results are shown in Figures 8 and 9.

The results of Figure 8 confirm earlier studies that the mouse antibody BB-1 does not compete with P16C10. These results also show that there is some cross-reactivity to L307.4 of approximately 50%. The results of Figure 8 confirm that BB-1 and L307.4 both compete with each other and that BB-1 completely blocks binding of CTLA-4Ig-Biotin to B7.1 transfected CHO cells. BB-1 does not significantly affect P16C10 binding to B7.1 positive CHO cells.

The results shown in Figure 9 indicate better than 50% competition when CTLA-4Ig-Biotin is used in the binding experiment. Figure 9 shows that CTLA-4Ig-Biotin is effectively blocked by all B7.1 inhibitors except P16C10, therefore P16C10 recognizes a unique binding determinate on B7.1 which allows the normal CTLA-4 ligand binding in the generation of negative signals. Earlier functional studies (data not shown) suggest a weakened ability of L307.4 to block IL-2 production in allogeneic MLR, which correlates with the hypothesis that it may interfere with CTLA-4 negative signaling. It is not clear how many of the other murine antibodies reported in the literature give complete inhibition of CTLA-4 binding; however, this issue may be important in defining the true functional mechanisms of B7.1 and B7.2 specific antibodies.

These results confirm earlier studies using B7-Ig in competition with P16C10-Biotin for binding to B7.1 transfected CHO cells. The studies also confirm earlier observations of no inhibition of the P16C10 by CTLA-4Ig. These results are highly suggestive that the primate antibodies are specific for a unique B7.1 epitope independent of the CTLA-4 binding site which interacts primarily with CD28. This type of interaction would provide a benefit, since it has the ability to block binding of B7.1 to CD28 receptors while still allowing the negative signaling function of CTLA-4 to occur uninhibited. This perceived interaction may lead to a down regulation of the overall T cell activation response regardless of the predominance of either Th1 or Th2 phenotypes.

Similar results were obtained using P7C10 when it was substituted for P16C10 in the experiments (data not provided).

### Example 11

### Experiment demonstrating the ability of P16C10 to bind and block B7.1 interactions with CD28 receptor.

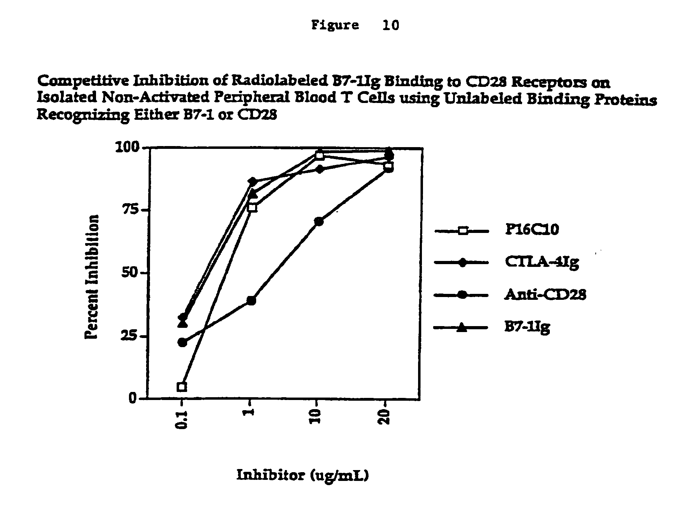

An experiment to determine if P16C10 binding of B7.1 can block the interaction of B7.1 with CD28 was attempted by radiolabeling B7.1Ig with ¹²⁵I, followed by competitive binding to CD28 positive non-activated peripheral blood T lymphocytes. The results shown in Figure 10 demonstrate that the radiolabeled B7.1Ig binds specifically to the T cells, as confirmed by inhibition with unlabeled B7.1Ig. The results also show that CTLA-4Ig, anti-CD28 and P16C10 are all capable of blocking this interaction. The results further confirm that P16C10 blocks binding of the CD28/B7 interaction with an IC₅₀ of < 1 ug/mL.

The above results were obtained under conditions where no membrane associated CTLA-4 was expressed (Linsley et al., J. Exp. Med. 173:721-730 (1991)) and confirmed by blocking with the anti-CD28 antibody.

Similar results were obtained using P7C10 when it was substituted for P16C10 in the experiments (data not provided).

It is expected that these primatized antibodies, given their probable low antigenicity and human effector function, will be well suited as therapeutics. In fact, it has recently been shown that primatized 16C10. exhibits human Clq binding.

Those skilled in the art will recognize or be able to ascertain using no more than routine experimentation many equivalents to the specific embodiments of the invention described herein.

## Claims

1. A monoclonal antibody or a fragment thereof that binds specifically to human CD80 antigen and inhibits the binding of the CD80 antigen to CD28 but does not inhibit the binding of the CD80 antigen to CTLA-4.

2. A monoclonal antibody or fragment thereof according to claim 1, wherein said antibody or fragment thereof binds to the same epitope on CD80 as :
an antibody having the light and heavy chain sequences set forth as SEQ ID NO:1 and SEQ ID NO:2, respectively ; or
an antibody having the light and heavy chain sequences set forth as SEQ ID NO:5 and SEQ ID NO:6, respectively.

3. A monoclonal antibody or fragment thereof according to claim 1, comprising monkey light and heavy chain variable regions and monkey light and heavy chain constant regions.

4. A monoclonal antibody or fragment thereof according to claim 1, comprising monkey light and heavy chain variable regions and human light and heavy chain constant regions.

5. A monoclonal antibody or fragment thereof according to claim 3 or claim 4, which comprises the light and heavy chain variable regions of monoclonal monkey antibody 7C10 with the amino acid sequences shown in SEQ ID NO:1 and SEQ ID NO:2, respectively.

6. A monoclonal antibody or fragment thereof according to claim 3 or claim 4, which comprises the light and heavy chain variable regions of monoclonal monkey antibody 16C10 with the amino acid sequences shown in SEQ ID NO: 5 and SEQ ID NO:6, respectively.

7. A monoclonal antibody or fragment thereof according to claim 4, which comprises a human heavy chain constant region selected from human gamma 1 constant region, human gamma 4 constant region, and human gamma 4 PE constant region.

8. A monoclonal antibody fragment according to any one of the preceding claims, which is selected from the group consisting of Fab, F(ab')₂, and Fv.

9. A monoclonal antibody or fragment thereof according to any of claims 1 to 8, which inhibits the production of IL-2 by T lymphocytes *in vitro.*

10. A monoclonal antibody or fragment thereof according to claim 9, which inhibits IL-2 production by T lymphocytes *in vitro* when present at a concentration of at least 10 µg/ml.

11. Use of a monoclonal antibody or fragment thereof according to any one of claims 1 to 8 in the preparation of a medicament for preventing or treating a disease or disorder by inhibiting interaction between T cells and B cells via the CD80/CD28 pathway, wherein said disease or disorder is selected from the group consisting of rejection of a transplanted organ or tissue, graft versus host disease, autoimmune, inflammatory, proliferative and hyperproliferative diseases, infectious disease, allergies, cutaneous manifestations of immunologically mediated diseases, reversible obstructive airways disease, and B cell lymphoma.

12. Use according to claim 11, wherein said medicament is for preventing or treating graft versus host disease or rejection of a transplanted organ or tissue, wherein the graft or transplanted organ or tissue is selected from kidney, heart, lung, bone marrow, skin, and cornea.

13. Use according to claim 11, wherein said medicament is for preventing or treating an autoimmune or inflammatory disease or disorder.

14. Use according to claim 11, wherein said medicament is for preventing or treating a disease or disorder selected from the group consisting of rheumatoid arthritis, lupus erythematosus, systemic lupus erythematosus, Hashimoto's thyroiditis, multiple sclerosis, myasthenia gravis, aplastic anemia, type 1 diabetes, uveitis, nephrotic syndrome, psoriasis, atopical dermatitis, contact dermatitis, eczematous dermatitis, seborrheic dermatitis, lichen planus, pemphigus, bullous pemphigus, epidermolysis bullosa, urticaria, angioedemas, vasculitides, erythema, cutaneous eosinophilias, Alopecia areata, migraine, eczema, rhinitis, coeliac disease, proctitis, eosinophilia gastroenteritis, mastocytosis, Crohn's disease and ulcerative colitis, and idiopathic thrombocytopenic purpura.

15. Use according to claim 11, wherein said disease or disorder is selected from the group consisting of idiopathic thrombocytopenia purpura, systemic lupus erythematosus, type 1 diabetes mellitus, multiple sclerosis, aplastic anemia, psoriasis, allergy, inflammatory bowel disease, and rheumatoid arthritis.

16. Use according to claim 11, wherein said medicament is for treating HIV infection.

17. Use of a monoclonal antibody or fragment thereof as defined in any one of claims 1 to 10 in the preparation of a medicament for use in combination with
a) an agent selected from the group consisting of IL-7, IL-10, CTLA4-Ig, soluble CTLA-4, anti-CD28 antibodies, and fragments of anti-CD28 antibodies that modulate the CD80/:CD28 pathway; or
b) a small molecule immunosuppressant selected from the group consisting of cyclosporin A, FK506, anti-TNF-α antibodies, anti-CD54 antibodies, anti-CD11 antibodies, anti-CD11a antibodies, and anti-IL-1 antibodies for preventing or treating a disease or disorder by inhibiting interaction between T cells and B cells via the CD80/CD28 pathway, wherein said disease or disorder is selected from the group consisting of rejection of a transplanted organ or tissue, graft versus host disease, autoimmune, inflammatory, proliferative and hyperproliferative diseases, infectious disease, allergies, cutaneous manifestations of immunologically mediated diseases, reversible obstructive airways disease, and B cell lymphoma.

18. A pharmaceutical composition suitable for preventing or treating a disease or disorder by inhibiting interaction between T cells and B cells via the CD80:CD28 pathway, which composition comprises a prophylactically or therapeutically effective amount of a monoclonal antibody or fragment thereof according to any of claims 1 to 9, and a pharmaceutically acceptable carrier.

19. Use according to claim 17, wherein said disease or disorder is graft versus host disease or rejection of a transplanted organ or tissue, wherein the graft or transplanted organ or tissue is selected from kidney, heart, lung, bone marrow, skin, and cornea.

20. Use according to claim 17, wherein said disease or disorder is an autoimmune or inflammatory disease or disorder.

21. Use according to claim 17, wherein said disease or disorder is selected from the group consisting of rheumatoid arthritis, lupus erythematosus, systemic lupus erythematosus, Hashimoto's thyroiditis, multiple sclerosis, myasthenia gravis, aplastic anemia, type I diabetes, uveitis, nephrotic syndrome, psoriasis, atopical dermatitis, contact dermatitis, eczematous dermatitis, seborrheic dermatitis, lichen planus, pemphigus, bullous pemphigus, epidermolysis bullosa, urticaria, angioedemas, vasculitides, erythema, cutaneous eosinophilias, Alopecia areata, migraine, eczema, rhinitis, coeliac disease, proctitis, eosinophilia gastroenteritis, mastocytosis, Crohn's disease and ulcerative colitis, and idiopathic thrombocytopenic purpura.

22. Use according to claim 17, wherein said disease or disorder is selected from the group consisting of idiopathic thrombocytopenia purpura, systemic lupus erythematosus, type 1 diabetes mellitus, multiple sclerosis, aplastic anemia, psoriasis, allergy, inflammatory bowel disease, and rheumatoid arthritis.

23. Use according to claim 17, wherein said disease or disorder is associated with HIV infection.

24. Use according to claim 11, wherein said disease or disorder is a B cell lymphoma.

25. Use according to claim 11 of a monoclonal antibody or fragment thereof according to claim 2, wherein the monoclonal antibody or fragment thereof binds to the same epitope on CD80 as an antibody having the light and heavy chain sequences set forth as SEQ ID NO: 5 and SEQ ID NO: 6, respectively.

26. Use according to claim 11 of a monoclonal antibody according to claim 2, wherein the monoclonal antibody binds to the same epitope on CD80 as an antibody having the light and heavy chain sequences set forth as SEQ ID NO: 5 and SEQ ID NO: 6, respectively, and said monoclonal antibody comprises monkey light and heavy chain variable regions and human light and heavy chain constant regions.

27. Use according to claim 17, wherein said disease or disorder is a B cell lymphoma.

28. Use according to claim 17 of a monoclonal antibody or fragment thereof according to claim 2, wherein the monoclonal antibody or fragment thereof binds to the same epitope on CD80 as an antibody having the light and heavy chain sequences set forth as SEQ ID NO: 5 and SEQ ID NO: 6, respectively.

29. Use according to claim 17 of a monoclonal antibody according to claim 2, wherein the monoclonal antibody binds to the same epitope on CD80 as an antibody having the light and heavy chain sequences set forth as SEQ ID NO: 5 and SEQ ID NO: 6, respectively, and said monoclonal antibody comprises monkey light and heavy chain variable regions and human light and heavy chain constant regions.

30. A pharmaceutical composition according to claim 18 for preventing or treating a B cell lymphoma.

31. A pharmaceutical composition according to claim 18 comprising a monoclonal antibody or fragment thereof according to claim 2, wherein the monoclonal antibody or fragment thereof binds to the same epitope on CD80 as an antibody having the light and heavy chain sequences set forth as SEQ ID NO: 5 and SEQ ID NO: 6, respectively.

32. A pharmaceutical composition according to claim 18 comprising a monoclonal antibody according to claim 2, wherein the monoclonal antibody binds to the same epitope on CD80 as an antibody having the light and heavy chain sequences set forth as SEQ ID NO: 5 and SEQ ID NO: 6, respectively, and said monoclonal antibody comprises monkey light and heavy chain variable regions and human light and heavy chain constant regions.

33. A monoclonal antibody according to claim 2, wherein the monoclonal antibody binds to the same epitope on CD80 as an antibody having the light and heavy chain sequences set forth as SEQ ID NO: 5 and SEQ ID NO: 6, respectively, and wherein said monoclonal antibody comprises monkey light and heavy chain variable regions and human light and heavy chain constant regions.

## Patentansprüche

1. Monoklonaler Antikörper oder Fragment davon, welche spezifisch an humanes CD80-Antigen binden und das Binden des CD80-Antigens an CD28 inhibieren, welche aber nicht das Binden des CD80-Antigens an CTLA-4 inhibieren.

2. Monoklonaler Antikörper oder Fragment davon gemäß Anspruch 1, wobei der Antikörper oder das Fragment davon an das gleiche Epitop an CD80 binden wie:
ein Antikörper mit den leichten und schweren Kettensequenzen, welche als SEQ ID Nr.: 1 beziehungsweise SEQ ID Nr.: 2 dargelegt sind; oder
ein Antikörper mit den leichten und schweren Kettensequenzen, welche als SEQ ID Nr.: 5 beziehungsweise SEQ ID Nr.: 6 dargelegt sind.

3. Monoklonaler Antikörper oder Fragment davon gemäß Anspruch 1, umfassend leichte und schwere variable Affen-Kettenregionen und leichte und schwere konstante Affen-Kettenregionen.

4. Monoklonaler Antikörper oder Fragment davon gemäß Anspruch 1, umfassend leichte und schwere variable Affen-Kettenregionen und leichte und schwere konstante humane Kettenregionen.

5. Monoklonaler Antikörper oder Fragment davon gemäß Anspruch 3 oder Anspruch 4, welche die leichten und schweren variablen Kettenregionen des monoklonalen Affen-Antikörpers 7C10 mit den in SEQ ID Nr.: 1 beziehungsweise SEQ ID Nr.: 2 gezeigten Aminosäuresequenzen umfassen.

6. Monoklonaler Antikörper oder Fragment davon gemäß Anspruch 3 oder Anspruch 4, welche die leichten und schweren variablen Kettenregionen des monoklonalen Affen-Antikörpers 16C10 mit den in SEQ ID Nr.: 5 beziehungsweise SEQ ID Nr.: 6 gezeigten Aminosäuresequenzen umfassen.

7. Monoklonaler Antikörper oder Fragment davon gemäß Anspruch 4, welche eine humane schwere konstante Kettenregion, ausgewählt aus humaner konstanter Gamma 1-Region, humaner konstanter Gamma 4-Region und humaner konstanter Gamma 4-PE-Region, umfassen.

8. Monoklonales Antikörperfragment gemäß einem der vorhergehenden Ansprüche, welches aus der Gruppe ausgewählt ist, die aus Fab, F(ab')₂ und Fv besteht.

9. Monoklonaler Antikörper oder Fragment davon gemäß einem der Ansprüche 1 bis 8, welche die Herstellung von IL-2 durch T-Lymphozyten *in vitro* inhibieren.

10. Monoklonaler Antikörper oder Fragment davon gemäß Anspruch 9, welche die IL-2-Herstellung durch T-Lymphozyten *in vitro* inhibieren, wenn sie in einer Konzentration von mindestens 10 µg/ml vorhanden sind.

11. Verwendung eines monoklonalen Antikörpers oder eines Fragments davon gemäß einem der Ansprüche 1 bis 8 zur Herstellung eines Medikaments zur Vorbeugung oder Behandlung einer Erkrankung oder Störung durch Inhibieren der Wechselwirkung zwischen T-Zellen und B-Zellen über den CD80/CD28-Weg, wobei die Erkrankung oder Störung aus der Gruppe ausgewählt ist, welche aus Abstoßung eines transplantierten Organs oder Gewebes, Transplantat-Wirt-Reaktion, Autoimmun-, Entzündungs-, proliferativen und hyperproliferativen Erkrankungen, Infektionserkrankung, Allergien, kutanen Manifestationen von durch das Immunsystem vermittelten Erkrankungen, reversibler obstruktiver Atemwegserkrankung und B-Zellen-Lymphom besteht.

12. Verwendung gemäß Anspruch 11, wobei das Medikament zur Vorbeugung oder Behandlung von Transplantat-Wirt-Reaktion oder Abstoßung eines transplantierten Organs oder Gewebes ist, wobei das Transplantat oder transplantierte Organ oder Gewebe aus Niere, Herz, Lunge, Knochenmark, Haut und Kornea ausgewählt sind.

13. Verwendung gemäß Anspruch 11, wobei das Medikament zur Vorbeugung oder Behandlung einer Autoimmun- oder Entzündungserkrankung oder -störung ist.

14. Verwendung gemäß Anspruch 11, wobei das Medikament zur Vorbeugung oder Behandlung einer Erkrankung oder Störung, welche aus der Gruppe ausgewählt sind, die aus rheumatoider Arthritis, Lupus erythematodes, systemischen Lupus erythematodes, Hashimoto-Thyreoiditis, Multipler Sklerose, Myasthenia gravis, aplastischer Anämie, Diabetes Typ 1, Uveitis, nephrotischem Syndrom, Psoriasis, atopischer Dermatitis, Kontaktdermatitis, ekzematösen Hautentzündungen, seborrhoischer Dermatitis, Lichen planus, Pemphigus, Alterspemphigus, Epidermolysis bullosa, Urtikaria, angioneurotischen Ödemen, Gefäßentzündungen, Erythem, kutanen Eosinophilien, Alopecia areata, Migräne, Ekzem, Rhinitis, Zöliakie, Proktitis, eosinophiler Gastroenteritis, Mastozytose, Morbus Crohn und Colitis ulcerosa und idiopathischer thrombozytopenischer Purpura besteht, ist.

15. Verwendung gemäß Anspruch 11, wobei die Erkrankung oder Störung aus der Gruppe ausgewählt ist, welche aus idiopathischer thrombozytopenischer Purpura, systemischen Lupus erythematodes, Diabetes mellitus Typ 1, Multipler Sklerose, aplastischer Anämie, Psoriasis, Allergie, entzündlicher Darmerkrankung und rheumatoider Arthritis besteht.

16. Verwendung gemäß Anspruch 11, wobei das Medikament zur Behandlung von HIV-Infektion ist.

17. Verwendung eines monoklonalen Antikörpers oder eines Fragments davon, wie in einem der Ansprüche 1 bis 10 definiert, zur Herstellung eines Medikaments zur aufeinanderfolgenden Verwendung in Kombination mit
a) einem Mittel, welches aus der Gruppe ausgewählt ist, die aus IL-7, IL-10, CTLA4-Ig, löslichem CTLA-4, anti-CD28-Antikörpern und anti-CD28-Antikörper-Fragmenten, welche den CD80/CD28-Weg modulieren, besteht; oder
b) einem kleinen Immunsuppressionsmolekül, welches aus der Gruppe ausgewählt ist, die aus Cyclosporin A, FK506, anti-TNF-α-Antikörpern, anti-CD54-Antikörpern, anti-CD11-Antikörpern, anti-CD11a-Antikörpern und anti-IL-1-Antikörpern, besteht;
zur Vorbeugung oder Behandlung einer Erkrankung oder Störung durch Inhibieren der Wechselwirkung zwischen T-Zellen und B-Zellen über den CD80/CD28-Weg, wobei die Erkrankung oder Störung aus der Gruppe ausgewählt ist, welche aus Abstoßung eines transplantierten Organs oder Gewebes, Transplantat-Wirt-Reaktion, Autoimmun-, Entzündungs-, proliferativen und hyperproliferativen Erkrankungen, Infektionserkrankung, Allergien, kutanen Manifestationen von durch das Immunsystem vermittelten Erkrankungen, reversibler obstruktiver Atemwegserkrankung und B-Zellen-Lymphom besteht.

18. Arzneimittel, welches zur Vorbeugung oder Behandlung einer Erkrankung oder Störung durch Inhibierung der Wechselwirkung zwischen T-Zellen und B-Zellen über den CD80/CD28-Weg geeignet ist, wobei die Zusammensetzung eine prophylaktisch oder therapeutisch wirksame Menge eines monoklonalen Antikörpers oder eines Fragments davon gemäß einem der Ansprüche 1 bis 9 und einen pharmazeutisch verträglichen Träger umfasst.

19. Verwendung gemäß Anspruch 17, wobei die Erkrankung oder Störung Transplantat-Wirt-Reaktion oder Abstoßung eines transplantierten Organs oder Gewebes ist, wobei das Transplantat oder transplantierte Organ oder Gewebe aus Niere, Herz, Lunge, Knochenmark, Haut und Kornea ausgewählt sind.

20. Verwendung gemäß Anspruch 17, wobei die Erkrankung oder Störung eine Autoimmun- oder Entzündungserkrankung oder -störung ist.

21. Verwendung gemäß Anspruch 17, wobei die Erkrankung oder Störung aus der Gruppe ausgewählt sind, die aus rheumatoider Arthritis, Lupus erythematodes, systemischen Lupus erythematodes, Hashimoto-Thyreoiditis, Multipler Sklerose, Myasthenia gravis, aplastischer Anämie, Diabetes Typ 1, Uveitis, nephrotischem Syndrom, Psoriasis, atopischer Dermatitis, Kontaktdermatitis, ekzematösen Hautentzündungen, seborrhoischer Dermatitis, Lichen planus, Pemphigus, Alterspemphigus, Epidermolysis bullosa, Urtikaria, angioneurotischen Ödemen, Gefäßentzündungen, Erythem, kutanen Eosinophilien, Alopecia areata, Migräne, Ekzem, Rhinitis, Zöliakie, Proktitis, eosinophile Gastroenteritis, Mastozytose, Morbus Crohn und Colitis ulcerosa und idiopathischer thrombozytopenischer Purpura besteht.

22. Verwendung gemäß Anspruch 17, wobei die Erkrankung oder Störung aus der Gruppe ausgewählt ist, welche aus idiopathischer thrombozytopenischer Purpura, systemischen Lupus erythematodes, Diabetes mellitus Typ 1, Multipler Sklerose, aplastischer Anämie, Psoriasis, Allergie, entzündlicher Darmerkrankung und rheumatoider Arthritis besteht.

23. Verwendung gemäß Anspruch 17, wobei die Erkrankung oder Störung in Zusammenhang mit HIV-Infektion steht.

24. Verwendung gemäß Anspruch 11, wobei die Erkrankung oder Störung ein B-Zellen-Lymphom ist.

25. Verwendung gemäß Anspruch 11 eines monoklonalen Antikörpers oder eines Fragments davon gemäß Anspruch 2, wobei der monoklonale Antikörper oder das Fragment davon an das gleiche Epitop an CD80 wie ein Antikörper mit den leichten und schweren Kettensequenzen, welche als SEQ ID Nr.: 5 beziehungsweise SEQ ID Nr.: 6 dargelegt sind, binden.

26. Verwendung gemäß Anspruch 11 eines monoklonalen Antikörpers gemäß Anspruch 2, wobei der monoklonale Antikörper an das gleiche Epitop an CD80 wie ein Antikörper mit den leichten und schweren Kettensequenzen, welche als SEQ ID Nr.: 5 beziehungsweise SEQ ID Nr.: 6 dargelegt sind, bindet und wobei der monoklonale Antikörper leichte und schwere variable Affen-Kettenregionen und leichte und schwere konstante humane Kettenregionen umfasst.

27. Verwendung gemäß Anspruch 17, wobei die Erkrankung oder Störung ein B-Zellen-Lymphom ist.

28. Verwendung gemäß Anspruch 17 eines monoklonalen Antikörpers oder eines Fragments davon gemäß Anspruch 2, wobei der monoklonale Antikörper oder das Fragment davon an das gleiche Epitop an CD80 wie ein Antikörper mit den leichten und schweren Kettensequenzen, welche als SEQ ID Nr.: 5 beziehungsweise SEQ ID Nr.: 6 dargelegt sind, binden.

29. Verwendung gemäß Anspruch 17 eines monoklonalen Antikörpers gemäß Anspruch 2, wobei der monoklonale Antikörper an das gleiche Epitop an CD80 wie ein Antikörper mit den leichten und schweren Kettensequenzen, welche als SEQ ID Nr.: 5 beziehungsweise SEQ ID Nr.: 6 dargelegt sind, bindet und wobei der monoklonale Antikörper leichte und schwere variable Affen-Kettenregionen und leichte und schwere konstante humane Kettenregionen umfasst.

30. Arzneimittel gemäß Anspruch 18 zur Vorbeugung oder Behandlung eines B-Zellen-Lymphoms.

31. Arzneimittel gemäß Anspruch 18, umfassend einen monoklonalen Antikörper oder ein Fragment davon gemäß Anspruch 2, wobei der monoklonale Antikörper oder das Fragment davon an das gleiche Epitop an CD80 wie ein Antikörper mit den leichten und schweren Kettensequenzen, welche als SEQ ID Nr.: 5 beziehungsweise SEQ ID Nr.: 6 dargelegt sind, binden.

32. Arzneimittel gemäß Anspruch 18, umfassend einen monoklonalen Antikörper gemäß Anspruch 2, wobei der monoklonale Antikörper an das gleiche Epitop an CD80 wie ein Antikörper mit den leichten und schweren Kettensequenzen, welche als SEQ ID Nr.: 5 beziehungsweise SEQ ID Nr.: 6 dargelegt sind, bindet und wobei der monoklonale Antikörper leichte und schwere variable Affen-Kettenregionen und leichte und schwere konstante humane Kettenregionen umfasst.

33. Monoklonaler Antikörper gemäß Anspruch 2, wobei der monoklonale Antikörper an das gleiche Epitop an CD80 wie ein Antikörper mit den leichten und schweren Kettensequenzen, welche als SEQ ID Nr.: 5 beziehungsweise SEQ ID Nr.: 6 dargelegt sind, bindet und wobei der monoklonale Antikörper leichte und schwere variable Affen-Kettenregionen und leichte und schwere konstante humane Kettenregionen umfasst.

## Revendications

1. Anticorps monoclonal ou fragment de celui-ci qui se lie spécifiquement à l'antigène CD80 humain et qui inhibe la liaison de l'antigène CD80 à CD28 mais qui n'inhibe pas la liaison de l'antigène CD80 à CTLA-4.

2. Anticorps monoclonal ou fragment de celui-ci selon la revendication 1, ledit anticorps ou fragment de celui-ci se liant au même épitope sur CD80 que :
un anticorps ayant les séquences de chaîne légère et lourde décrites dans SEQ ID N° 1 et SEQ ID N° 2, respectivement ; ou
un anticorps ayant les séquences de chaîne légère et lourde décrites dans SEQ ID N° 5 et SEQ ID N° 6, respectivement.

3. Anticorps monoclonal ou fragment de celui-ci selon la revendication 1, comprenant des régions variables de chaîne légère et lourde de singe et des régions constantes de chaîne légère et lourde de singe.

4. Anticorps monoclonal ou fragment de celui-ci selon la revendication 1, comprenant des régions variables de chaîne légère et lourde de singe et des régions constantes de chaîne légère et lourde humaine.

5. Anticorps monoclonal ou fragment de celui-ci selon la revendication 3 ou la revendication 4, qui comprend les régions variables de chaîne légère et lourde d'anticorps monoclonal de singe 7C10 ayant les séquences d'acides aminés présentées dans SEQ ID N° 1 et SEQ ID N° 2, respectivement.

6. Anticorps monoclonal ou fragment de celui-ci selon la revendication 3 ou la revendication 4, qui comprend les régions variables de chaîne légère et lourde d'anticorps monoclonal de singe 16C10 ayant les séquences d'acides aminés présentées dans SEQ ID N° 5 et SEQ ID N° 6, respectivement.

7. Anticorps monoclonal ou fragment de celui-ci selon la revendication 4, qui comprend une région constante de chaîne lourde humaine choisie parmi la région constante gamma 1 humaine, la région constante gamma 4 humaine, et la région constante gamma 4 PE humaine.

8. Fragment d'anticorps monoclonal selon l'une quelconque des revendications précédentes, qui est choisi dans le groupe consistant en Fab, F(ab')₂, et Fv.

9. Anticorps monoclonal ou fragment de celui-ci selon l'une quelconque des revendications 1 à 8, qui inhibe la production d'IL-2 par les lymphocytes T *in vitro.*

10. Anticorps monoclonal ou fragment de celui-ci selon la revendication 9, qui inhibe la production d'IL-2 par les lymphocytes T *in vitro* lorsqu'il est présent à une concentration d'au moins 10 µg/ml.

11. Utilisation d'un anticorps monoclonal ou d'un fragment de celui-ci selon l'une quelconque des revendications 1 à 8 dans la préparation d'un médicament pour prévenir ou traiter une maladie ou un trouble en inhibant l'interaction entre les lymphocytes T et les lymphocytes B par la voie CD80/CD28, ladite maladie ou ledit trouble étant choisi dans le groupe consistant en le rejet d'un organe transplanté ou d'un tissu greffé, la maladie du greffon contre l'hôte, des maladies auto-immunes, inflammatoires, prolifératrices et hyperprolifératrices, une maladie infectieuse, les allergies, les manifestations cutanées de maladies traitées immunologiquement, les maladies obstructives réversibles des voies respiratoires et le lymphome à cellules B.

12. Utilisation selon la revendication 11, dans laquelle ledit médicament est destiné à prévenir ou à traiter la maladie du greffon contre l'hôte ou le rejet d'un organe transplanté ou d'un tissu greffé, ledit greffon ou ledit organe transplanté ou ledit tissu greffé étant choisi parmi le rein, le coeur, le poumon, la moelle osseuse, la peau et la cornée.

13. Utilisation selon la revendication 11, dans laquelle ledit médicament est destiné à prévenir ou à traiter une maladie ou un trouble auto-immun ou inflammatoire.

14. Utilisation selon la revendication 11, dans laquelle ledit médicament est destiné à prévenir ou à traiter une maladie ou un trouble choisi dans le groupe consistant en la polyarthrite rhumatoïde, le lupus érythémateux, le lupus érythémateux systémique, la thyroïdite d'Hashimoto, la sclérose en plaques, la myasthénie grave, l'anémie aplastique, le diabète de type 1, l'uvéite, le syndrome néphrotique, le psoriasis, la dermatite atopique, la dermatite de contact, la dermatite eczémateuse, la dermatite séborrhéique, le lichen plan, le pemphigus, le pemphigus bulleux, l'épidermolyse bulleuse, l'urticaire, l'angio-oedème, les vascularites, l'érythème, l'éosinophilie cutanée, la pelade, la migraine, l'eczéma, la rhinite, la maladie coeliaque, la proctite, la gastroentérite à éosinophiles, la mastocytose, la maladie de Crohn et la rectocolite hémorragique, et le purpura thrombocytopénique idiopathique.

15. Utilisation selon la revendication 11, dans laquelle ladite maladie ou ledit trouble est choisi dans le groupe consistant en le purpura thrombocytopénique idiopathique, le lupus érythémateux systémique, le diabète sucré de type 1, la sclérose en plaques, l'anémie aplastique, le psoriasis, l'allergie, les maladies intestinales inflammatoires et la polyarthrite rhumatoïde.

16. Utilisation selon la revendication 11, dans laquelle ledit médicament est destiné à traiter l'infection par le VIH.

17. Utilisation d'un anticorps monoclonal ou d'un fragment de celui-ci comme défini dans l'une quelconque des revendications 1 à 10 dans la préparation d'un médicament pour utilisation en combinaison avec :
a) un agent choisi dans le groupe consistant en l'IL-7, l'IL-10, CTLA4-Ig, CTLA-4 soluble, des anticorps anti-CD28, et des fragments d'anticorps anti-CD28 qui modulent la voie CD80/CD28 ; ou
b) une petite molécule immunosuppressive choisie dans le groupe consistant en la cyclosporine A, FK506, les anticorps anti-TNF-α, les anticorps anti-CD54, les anticorps anti-CD11, les anticorps anti-CD11a, et les anticorps anti-IL-1 ;
pour prévenir ou traiter une maladie ou un trouble en inhibant l'interaction entre les cellules T et les cellules B *via* la voie CD80/CD28, ladite maladie ou ledit trouble étant choisi dans le groupe consistant en le rejet d'un organe transplanté ou d'un tissu greffé, la maladie du greffon contre l'hôte, des maladies auto-immunes, inflammatoires, prolifératrices et hyperprolifératrices, une maladie infectieuse, des allergies, les manifestations cutanées de maladies traitées immunologiquement, une maladie obstructive réversible des voies respiratoires et le lymphome à cellules B.

18. Composition pharmaceutique adaptée pour prévenir ou traiter une maladie ou un trouble en inhibant l'interaction entre les cellules T et les cellules B *via* la voie CD80/CD28, laquelle composition comprend une quantité efficace d'un point de vue prophylactique ou thérapeutique d'un anticorps monoclonal ou d'un fragment de celui-ci selon l'une quelconque des revendications 1 à 9, et un véhicule acceptable pharmaceutiquement.

19. Utilisation selon la revendication 17, dans laquelle ladite maladie ou ledit trouble est la maladie du greffon contre l'hôte ou le rejet d'un organe transplanté ou d'un tissu greffé, ledit greffon ou ledit organe transplanté ou ledit tissu greffé étant choisi parmi le rein, le coeur, le poumon, la moelle osseuse, la peau et la cornée.

20. Utilisation selon la revendication 17, dans laquelle ladite maladie ou ledit trouble est une maladie ou un trouble auto-immun ou inflammatoire.

21. Utilisation selon la revendication 17, dans laquelle ladite maladie ou ledit trouble est choisi dans le groupe consistant en la polyarthrite rhumatoïde, le lupus érythémateux, le lupus érythémateux systémique, la thyroïdite d'Hashimoto, la sclérose en plaques, la myasthénie grave, l'anémie aplastique, le diabète de type I, l'uvéite, le syndrome néphrotique, le psoriasis, la dermatite atopique, la dermatite de contact, la dermatite eczémateuse, la dermatite séborrhéique, le lichen plan, le pemphigus, le pemphigus bulleux, l'épidermolyse bulleuse, l'urticaire, les angio-oedèmes, les vascularites, l'érythème, l'éosinophilie cutanée, la pelade, la migraine, l'eczéma, la rhinite, la maladie coeliaque, la proctite, la gastroentérite à éosinophiles, la mastocytose, la maladie de Crohn et la rectocolite hémorragique, et le purpura thrombocytopénique idiopathique.

22. Utilisation selon la revendication 17, dans laquelle ladite maladie ou ledit trouble est choisi dans le groupe consistant en le purpura thrombocytopénique idiopathique, le lupus érythémateux systémique, le diabète sucré de type 1, la sclérose en plaques, l'anémie aplastique, le psoriasis, l'allergie, la maladie intestinale inflammatoire et la polyarthrite rhumatoïde.

23. Utilisation selon la revendication 17, dans laquelle ladite maladie ou ledit trouble est associé à l'infection par le VIH.

24. Utilisation selon la revendication 11, dans laquelle ladite maladie ou ledit trouble est un lymphome à cellules B.

25. Utilisation selon la revendication 11 d'un anticorps monoclonal ou d'un fragment de celui-ci selon la revendication 2, dans laquelle l'anticorps monoclonal ou son fragment se lie au même épitope sur CD80 qu'un anticorps ayant les séquences de chaîne légère et lourde présentées dans SEQ ID N°5 et SEQ ID N°6, respectivement.

26. Utilisation selon la revendication 11 d'un anticorps selon la revendication 2, dans laquelle l'anticorps monoclonal se lie au même épitope sur CD80 qu'un anticorps ayant les séquences de chaîne légère et lourde présentées dans SEQ ID N°5 et SEQ ID N°6, respectivement, et ledit anticorps monoclonal comprend des régions variables de chaîne légère et lourde de singe et des régions constantes de chaîne légère et lourde humaine.

27. Utilisation selon la revendication 17, dans laquelle ladite maladie ou ledit trouble est un lymphome à cellules B.

28. Utilisation selon la revendication 17 d'un anticorps monoclonal ou d'un fragment de celui-ci selon la revendication 2, dans laquelle l'anticorps monoclonal ou son fragment se lie au même épitope sur CD80 qu'un anticorps ayant les séquences de chaîne légère et lourde présentées dans SEQ ID N°5 et dans SEQ ID N°6, respectivement.

29. Utilisation selon la revendication 17 d'un anticorps monoclonal selon la revendication 2, dans laquelle l'anticorps monoclonal se lie au même épitope sur CD80 qu'un anticorps ayant les séquences de chaîne légère et lourde présentées dans SEQ ID N° 5 et SEQ ID N° 6, respectivement, et ledit anticorps monoclonal comprend des régions variables de chaîne légère et lourde de singe et des régions constantes de chaîne légère et lourde humaine.

30. Composition pharmaceutique selon la revendication 18 pour prévenir ou traiter un lymphome à cellules B.

31. Composition pharmaceutique selon la revendication 18 comprenant un anticorps monoclonal selon la revendication 2, dans laquelle l'anticorps monoclonal se lie au même épitope sur CD80 qu'un anticorps ayant les séquences de chaîne légère et lourde présentées dans SEQ ID N° 5 et SEQ ID N° 6, respectivement.

32. Composition pharmaceutique selon la revendication 18 comprenant un anticorps monoclonal ou un fragment de celui-ci selon la revendication 2, dans laquelle l'anticorps monoclonal ou son fragment se lient au même épitope sur CD80 qu'un anticorps ayant les séquences de chaîne légère et lourde présentées dans SEQ ID N° 5 et SEQ ID N° 6, respectivement, et ledit anticorps monoclonal comprend des régions variables de chaîne légère et lourde de singe et des régions constantes de chaîne légère et lourde humaine.

33. Anticorps monoclonal selon la revendication 2, ledit anticorps monoclonal se liant au même épitope sur CD80 qu'un anticorps ayant les séquences de chaîne légère et lourde présentées dans SEQ ID N° 5 et SEQ ID N° 6, respectivement, et ledit anticorps monoclonal comprenant des régions variables de chaîne légère et lourde de singe et des régions constantes de chaîne légère et lourde humaine.
